# EUROPÄISCHE PATENTSCHRIFT

(11) **EP 0 628 532 B1**
(45) Veröffentlichungstag und Bekanntmachung des Hinweises auf die Patenterteilung: **19.01.2000**
(21) Anmeldenummer: 94111142.9
(22) Anmeldetag: 03.09.1990
(51) Int. Cl.: C07C 43/225, C09K 19/30, C09K 19/12, C09K 19/34, C07C 25/18, C07D 213/64

(54) **Fluorbenzolderivate und flüssigkristallines Medium**
Fluorobenzole derivatives and mesomorphous medium
Dérivés de fluorobenzènes et milieu mesomorphe

(30) Priorität: 06.09.1989 DE 3929525; 06.09.1989 DE 3929526; 07.09.1989 DE 3929764; 28.03.1990 DE 4009907
(43) Veröffentlichungstag der Anmeldung: 14.12.1994
(62) Teilanmeldung aus: 90912861.3
(73) Patentinhaber: MERCK PATENT GmbH, 64293 Darmstadt (DE)
(72) Erfinder: Reiffenrath, Volker, D-64380 Rossdorf (DE); Kurmeier, Hans-Adolf, Dr., D-64342 Seeheim-Jugenheim (DE); Poetsch, Eike, Dr., D-64367 Mühltal (DE); Plach, Herbert, Dr., D-64287 Darmstadt (DE); Finkenzeller, Ulrich, Dr., D-68723 Plankstadt (DE); Bartmann, Ekkehard, Dr., D-64390 Erzhausen (DE); Krause, Joachim, Dr., D-64807 Dieburg (DE); Scheuble, Bernhaard, Dr., D-64342 Seeheim-Jugenheim (DE)

(56) Entgegenhaltungen:
- WO-A-89/02884
- WO-A-90/01056

## Beschreibung

Die Erfindung betrifft neue Fluorbenzolderivate der Formel I, 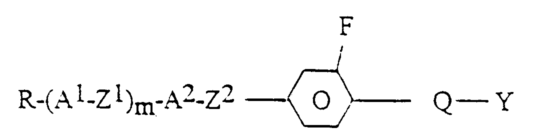 worin
- R: H, einen unsubstituierten, einen einfach durch CN oder CF₃ oder einen mindestens einfach durch Halogen substituierten Alkyl- oder Alkenylrest mit 1 bis 15 C-Atomen wobei in diesen Resten auch eine oder mehrere CH₂-Gruppen jeweils unabhängig voneinander durch -O-, -S-, 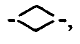 -CO-, -CO-O-, -O-CO-oder -O-CO-O- so ersetzt sein können, daß O-Atome nicht direkt miteinander verknüpft sind,
- A¹: einen
(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo (2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) durch CN oder Fluor substituiert sein können,
- Z¹: -CO-O-, O-CO-, -CH₂O-, -OCH₂-, -CH₂CH₂-, -CH=CH.-, -C≡C-, eine Einfachbindung, -(CH₂)₄- oder -CH=CH-CH₂CH₂-,
- m: 0, 1 oder 2,
- Y: F oder Cl,
- Q: -CF₂-, -OCF₂- oder -OCHF-, und
A²-Z² 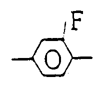 bedeutet.

Die Erfindung betrifft weiterhin die Verwendung dieser Verbindungen als Komponenten flüssigkristalliner Medien sowie Flüssigkristall- und elektrooptische Anzeigeelemente, die die erfindungsgemäßen flüssigkristallinen Medien enthalten.

Die Verbindungen der Formel I können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Medien geeignet sind und insbesondere gleichzeitig eine vergleichsweise geringe Viskosität besitzen sowie eine relativ hohe dielektrische Anisotropie.

Es wurde nun gefunden, daß Verbindungen der Formel I als Komponenten flüssigkristalliner Medien vorzüglich geeignet sind. Insbesondere verfügen sie über vergleichsweise niedrige Viskositäten. Mit ihrer Hilfe lassen sich stabile flüssig- kristalline Medien mit breitem Mesophasenbereich und vorteilhaften Werten für die optische und dielektrische Anisotropie erhalten. Diese Medien weisen ferner ein sehr gutes Tieftemperaturverhalten auf.

Flüssigkristalle der Formel 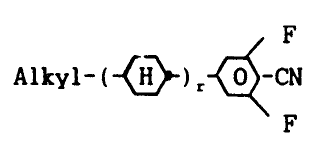 r = 1 oder 2 sind bereits aus DE 3209178 bekannt. Aus der JP 62-103057 sind Verbindungen der Formeln 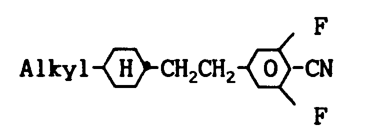 und 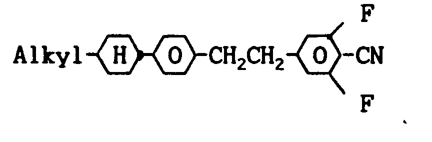 bekannt. In JP 63-216858 schließlich werden Verbindungen der Formel 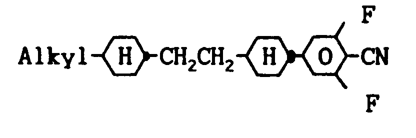 beschrieben. Aus den DE-OS 30 42 391 und DE-OS 31 39 130 sind Verbindungen der folgenden Formeln bekannt:

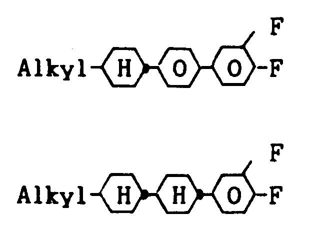

Verbindungen mit einer terminalen OCF₃- oder OCHF₂-Gruppe sind aus der WO 89/02884 bzw. WO 90/01056 bekannt. Die dort offenbarten

Verbindungen enthalten jedoch keine 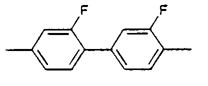 Einheit.

Verschiedene Verbindungen mit flüssigkristallinen Eigenschaften, in denen terminal eine CF₃-Gruppe gebunden ist, sind bereits bekannt (USP 4,330,426; USP 4,684,476; J.C. Liang and S. Kumar, Mol. Cryst. Liq. Cryst. 1987; Vol. 142, pp. 77-84). Diese Verbindungen haben jedoch oft einen stark smektogenen Charakter und sind für viele praktische Anwendungen weniger geeignet.

Im Hinblick auf die verschiedensten Einsatzbereiche derartiger Verbindungen mit hohem Δε war es jedoch wünschenswert, weitere Verbindungen zur Verfügung zu haben, die auf die jeweiligen Anwendungen genau maßgeschneiderte Eigenschaften aufweisen.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I sowie die Verwendung dieser Verbindungen als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, insbesondere elektroopische Anzeigeelemente, die derartige Medien enthalten.

Der Einfachheit halber bedeuten im folgenden A³ einen

Rest der Formel 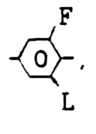 Cyc einen 1,4-Cyclohexylenrest,

Che einen 1,4-Cyclohexenylenrest, Dio einen 1,3-Dioxan-2,5-diylrest, Dit einen 1,3-Dithian-2,5-diylrest, Phe einen 1,4-Phenylenrest, Pyd einen Pyridin-2,5-diylrest, Pyr einen Pyrimidin-2,5-diylrest und Bi einen Bicyclo(2,2,2)-octylenrest, wobei Cyc und/oder Phe unsubstituiert oder ein- oder zweifach durch F oder CN substituiert sein können. L ist vorzugsweise F. Y ist vorzugsweise F.

A¹ ist vorzugsweise ausgewählt aus der Gruppe Cyc, Che, Phe, Pyr, Pyd und Dio.

Die Verbindungen der Formel I umfassen dementsprechend Verbindungen mit zwei Ringen der Teilformeln Ia und Ib:

R-A²-A³-Q-Y Ia

R-A²-Z²-A³-Q-Y Ib

Verbindungen mit drei Ringen der Teilformeln Ic bis If:

R-A¹-A²-A³-Q-Y Ic

R-A¹-Z¹-A²-Z²-A³-Q-Y Id

R-A¹-Z¹-A²-A³-Q-Y Ie

R-A¹-A²-Z²-A³-Q-Y If

sowie Verbindungen mit vier Ringen der Teilformeln Ig bis Im:

R-A¹-A¹-A²-A³-Q-Y Ig

R-A¹-Z¹-A¹-A²-A³-Q-Y Ih

R-A¹-A¹-Z¹-A²-A³-Q-Y Ii

R-A¹-A¹-A²-Z²-A³-Q-Y Ij

R-A¹-Z¹-A¹-Z¹-A²-A³-Q-Y Ik

R-A¹-A¹-Z¹-A²-Z²-A³-Q-Y Il

R-A¹-Z¹-A¹-Z¹-A²-Z²-A³-Q-Y Im

Darunter sind besonders diejenigen der Teilformeln Ia, Ib, Ic, Id, Ie, If, Ii und Il bevorzugt.

Die bevorzugten Verbindungen der Teilformel Ia umfassen diejenigen der Teilformeln Iaa:

R-Phe-A³-Q-Y Iaa

Die bevorzugten Verbindungen der Teilformel Ic umfassen diejenigen der Teilformeln

R-Phe-Phe-A³-Q-Y Ica

R-Pyd-Phe-A³-Q-Y Icg

R-Pyr-Phe-A³-Q-Y Ich

R-Cyc-Phe-A³-Q-Y Ick

R-Dio-Phe-A³-Q-Y Icm

R-Che-Phe-A³-Q-Y Icn

Darunter sind diejenigen der Formeln Ica besonders bevorzugt.

Die bevorzugten Verbindungen der Teilformel Id umfassen diejenigen der Teilformeln:

R-Phe-Z¹-Phe-Z²-A³-Q-Y Ida

R-Pyd-Z¹-Phe-Z²-A³-Q-Y Idf

R-Pyr-Z¹-Phe-Z²-A³-Q-Y Idh

R-Cyc-Z¹-Phe-Z²-A³-Q-Y Idk

R-Dio-Z¹-Phe-Z²-A³-Q-Y Idm

Die bevorzugten Verbindungen der Teilformel Ie umfassen diejenigen der Teilformeln:

R-Pyr-Z¹-Phe-A³-Q-Y Iea

R-Dio-Z¹-Phe-A³-Q-Y Ieb

R-Phe-Z¹-Phe-A³-Q-Y Iec

R-Cyc-Z¹-Phe-A³-Q-Y Ied

R-Pyd-Z¹-Phe-A³-Q-Y Iej

Die bevorzugten Verbindungen der Teilformel If umfassen diejenigen der Teilformeln:

R-Pyr-Phe-Z²-A³-Q-Y Ifa

R-Phe-Phe-Z²-A³-Q-Y Ifc

R-Pyd-Phe-Z²-A³-Q-Y Ifi

R-Dio-Phe-Z²-A³-Q-Y Ifj

R-Che-Phe-Z²-A³-Q-Y Ifn

R-Cyc-Phe-Z²-A³-Q-Y Ifp

In den Verbindungen der vor- und nachstehenden Formeln bedeutet Y vorzugsweise F.

R bedeutet vorzugsweise Alkyl, ferner Alkoxy. A¹ bedeutet bevorzugt Phe, Cyc, Che, Pyr oder Dio. Bevorzugt enthalten die Verbindungen der Formel I nicht mehr als einen der Reste Bi, Pyd, Pyr, Dio oder Dit.

Bevorzugt sind auch Verbindungen der Formel I sowie aller Teilformeln, in denen A¹ ein- oder zweifach durch F oder einfach durch CN substituiertes 1,4-Phenylen bedeutet. Insbesondere sind dies 2-Fluor-1,4-phenylen, 3-Fluor-1,4-phenylen und 3,5-Difluor-1,4-phenylen sowie 2-Cyan-1,4-phenylen und 3-Cyan-1,4-phenylen.

Z¹ bedeutet bevorzugt eine Einfachbindung, -CO-O-, -O-CO- und -CH₂CH₂-, in zweiter Linie bevorzugt -CH₂O- und -OCH₂-.

Falls einer der Reste Z¹ und Z² -(CH₂)₄- oder -CH=CH-CH₂CH₂- bedeutet, so ist der andere Rest Z¹ oder Z² (falls vorhanden) vorzugsweise die Einfachbindung.

m ist vorzugsweise 1 oder 0, insbesondere bevorzugt 0.

Falls R einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R einen Alkylrest bedeutet, in dem eine CH₂-Gruppe durch -O- und eine durch -CO- ersetzt ist, so sind diese bevorzugt benachbart. Somit beeinhalten diese eine Acyloxygruppe -CO-O- oder eine Oxycarbonylgruppe -O-CO-. Vorzugsweise sind diese geradkettig und haben 2 bis 6 C-Atome.

Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R einen Alkylrest bedeutet, in dem eine CH₂-Gruppe durch unsubstituiertes oder substituiertes -CH=CH- und eine benachbarte CH₂-Gruppe durch CO oder CO-O oder O-CO-ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxy∼propyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Falls R einen einfach durch CN oder CF₃ substituierten Alkyl- oder Alkenylrest bedeutet, so ist dieser Rest vorzugsweise geradkettig und die Substitution durch CN oder CF₃ in ω-Position.

Falls R einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sei, vorzugsweise jedoch in ω-Position.

Verbindungen der Formel I, die über für Polymerisationsreaktionen geeignete Flügelgruppen R verfügen, eignen sich zur Darstellung flüssigkristalliner Polymerer.

Verbindungen der Formeln I mit verzweigten Flügelgruppen R können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verbindungen der Formel I mit S_{A}-Phasen eignen sich beispielsweise für thermisch adressierte Displays.

Verzweigte Gruppen dieser Art enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R einen Alkylrest darstellt, in dem zwei oder mehr CH₂-Gruppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxy-ethyl, 3,3-Bis-carboxy-propyl, 4,4-Bis-carboxy-butyl, 5,5-Bis-carboxypentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxydecyl, Bis-(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxy-carbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-hexyl.

Verbindungen der Formel I, die über für Polykondensationen geeignete Flügelgruppen R verfügen, eignen sich zur Darstellung flüssigkristalliner Polykondensate.

Formel I umfaßt sowohl die Racemate dieser Verbindungen als auch die optischen Antipoden sowie deren Gemische.

Unter diesen Verbindungen der Formel I sowie den Unterformeln sind diejenigen bevorzugt, in denen mindestens einer der darin enthaltenden Reste eine der angegebenen bevorzugten Bedeutungen hat.

In den Verbindungen der Formel I sind diejenigen Stereoisomeren bevorzugt, in denen die Ringe Cyc und Piperidin trans-1,4-disubstituiert sind. Diejenigen der vorstehend genannten Formeln, die eine oder mehrere Gruppen Pyd, Pyr und/oder Dio enthalten, umschließen jeweils die beiden 2,5-Stellungsisomeren.

Die 1,4-Cyclohexenylen-Gruppe hat vorzugsweise folgende Strukturen:

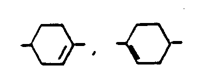

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart Bd IX, S. 867 ff.) beschrieben sind, und zwar unter Reaktionsbedingungen, die für die genannten Umsetzungen bekannt und geeignet sind.

Dabei kann man auch von an sich bekannten, hier nicht näher erwähnten Varianten Gebrauch machen.

Die erfindungsgemäßen Verbindungen können z.B. hergestellt, indem man eine Verbindung der Formel II, 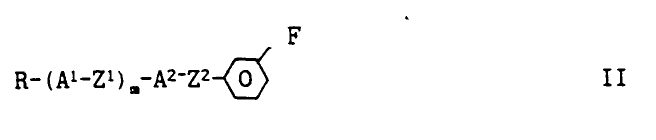 worin R, A¹, A², Z¹, Z² und m die angegebene Bedeutung haben, gemäß folgendem Reaktionsschema metalliert und anschließend mit einem geeigneten Elektrophil umsetzt:

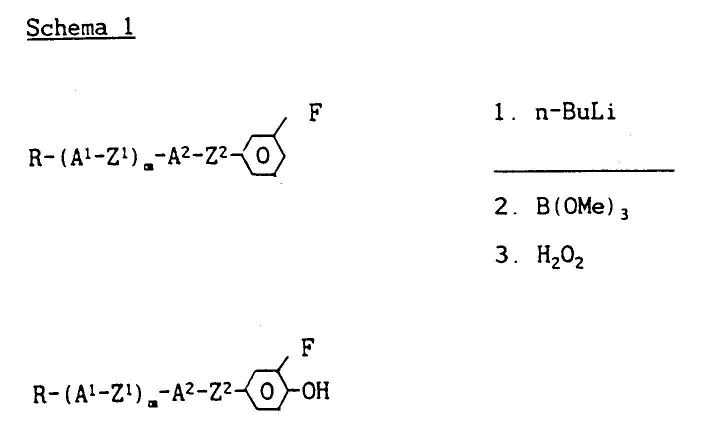

Aus dem erhaltenen Phenol sind die Zielprodukte mit Q = OCF₂ oder OCHF nach bekannten Methoden, z. B. durch Umsetzung mit Chlordifluormethan bzw. Tetrachlorkohlenstoff/HF erhältlich.

Weitere Synthesemethoden sind für den Fachmann augenscheinlich. Beispielsweise können in 5-Position entsprechend substituierte 1,3-Difluorbenzol-Verbindungen oder monofluorierte Analoga gemäß obigem Schema in die 2-OCF₂Y-1,3-difluor-Verbindungen oder monofluorierte Analoga (L = H) überführt werden und der Rest R-(A¹-Z¹)ₘ-A²-Z² anschließend durch in der Flüssigkristallchemie gebräuchliche Reaktionen (z.B. Veresterung, Veretherung oder Kopplungen z.B. gemäß der Artikel E. Poetsch, Kontakte (Darmstadt) 1988 (2), S. 15) eingeführt werden.

Weitere Synthesemethoden sind für den Fachmann augenscheinlich. Beispielsweise können in 5-Position entsprechend substituierte 1,3-Difluorbenzol-Verbindungen gemäß obigem Schema in die 2-Y-1,3-difluor-Verbindungen überführt werden und der Rest R-(A¹-Z¹)ₘ-A²-Z² anschließend durch in der Flüssigkristallchemie gebräuchliche Reaktionen (z.B. Veresterung, Veretherung oder Kopplungen z.B. gemäß der Artikel E. Poetsch, Kontakte (Darmstadt) 1988 (2), S. 15) angeführt werden.

Die erfindungsgemäßen Verbindungen mit L = H und Q-Y = CF₃ können hergestellt werden, indem man 3-Fluor-4-jodbrombenzol mit CF₃COONa in die Benzotrifluoridverbindung überführt und anschließend den Rest R-(A¹-Z¹)ₘ z.B. über übliche Kopplungsreaktionen einführt:

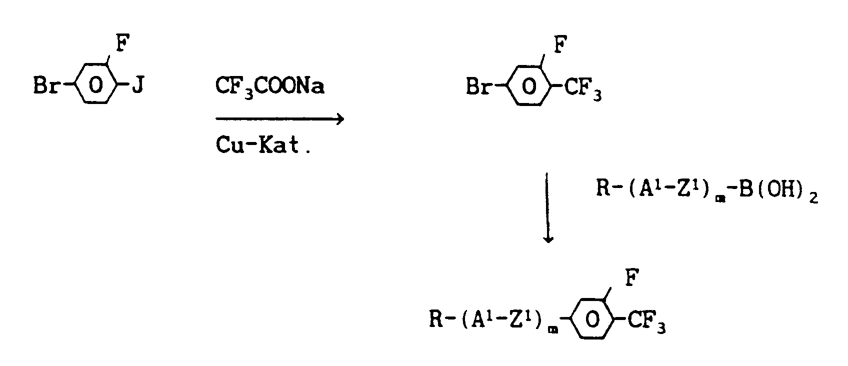

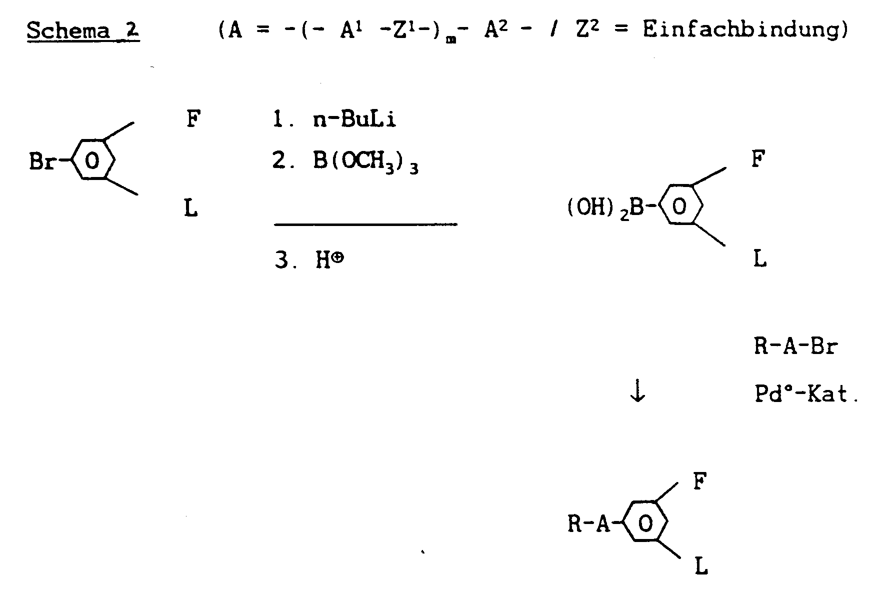

Die Ausgangsmaterialien sind entweder bekannt oder können in Analogie zu bekannten Verbindungen hergestellt werden.

Ester der Formel I können auch durch Veresterung entsprechender Carbonsäuren (oder ihrer reaktionsfähigen Derivate) mit Alkoholen bzw. Phenolen (oder ihren reaktionsfähigen Derivaten) oder nach der DCC-Methode (DCC = Dicyclohexylcarbodiimid) erhalten werden.

Die entsprechenden Carbonsäuren und Alkohole bzw. Phenole sind bekannt oder können in Analogie zu bekannten Verfahren hergestellt werden.

Die Synthese einiger besonders bevorzugter Verbindungen ist im folgenden näher angegeben:

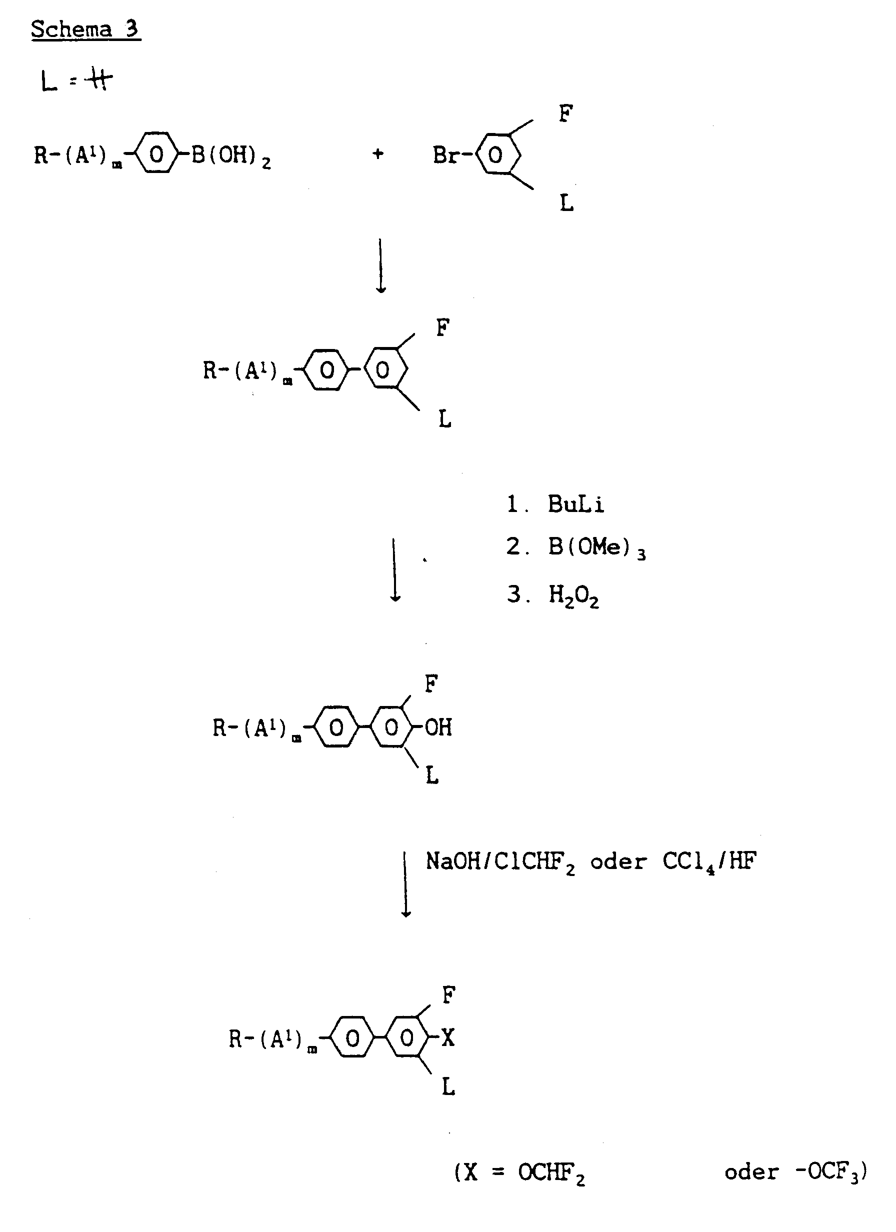

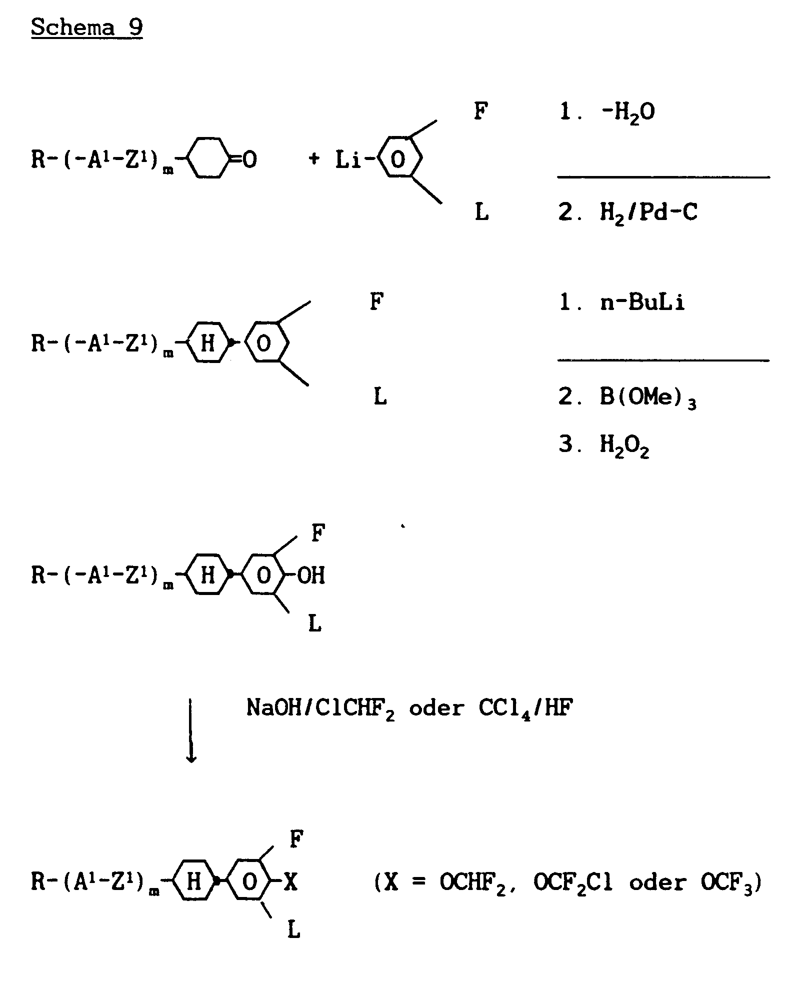

In Schema 3, ist vorzugsweise m 0 oder 1 und

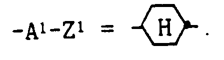

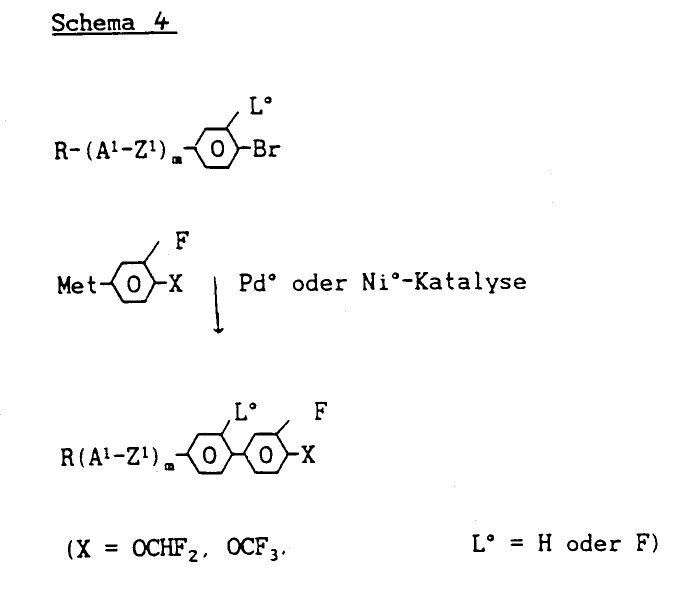

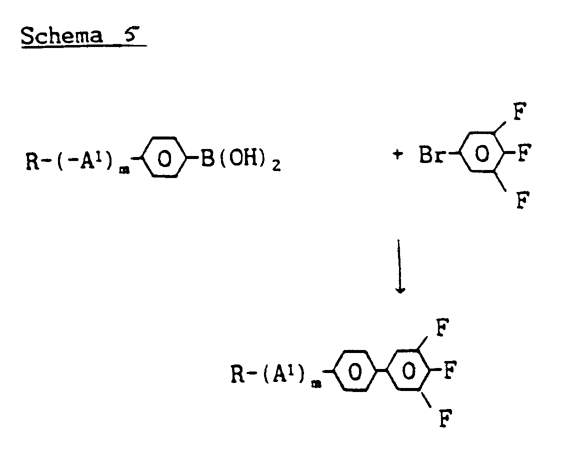

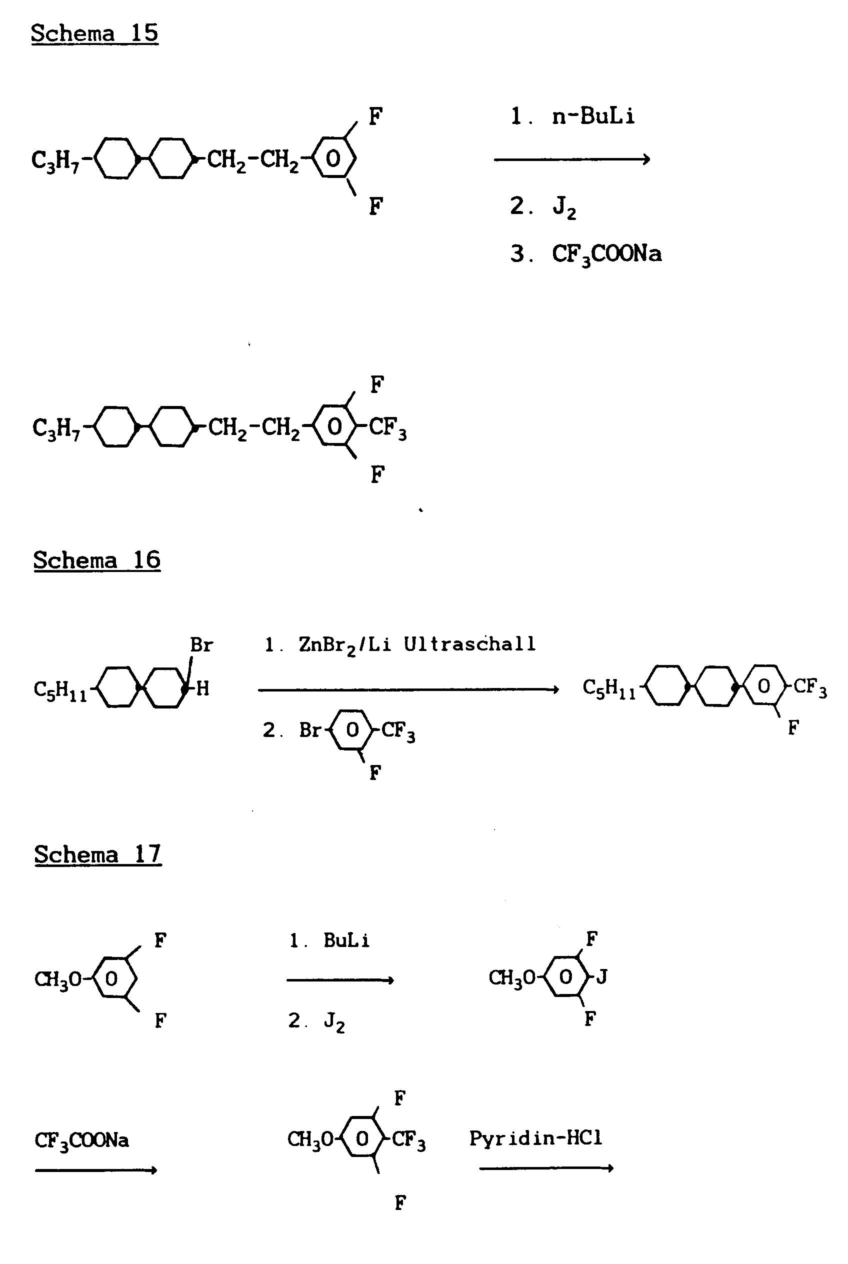

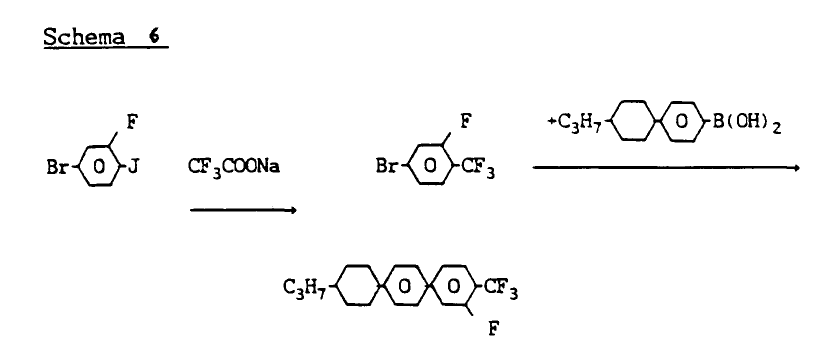

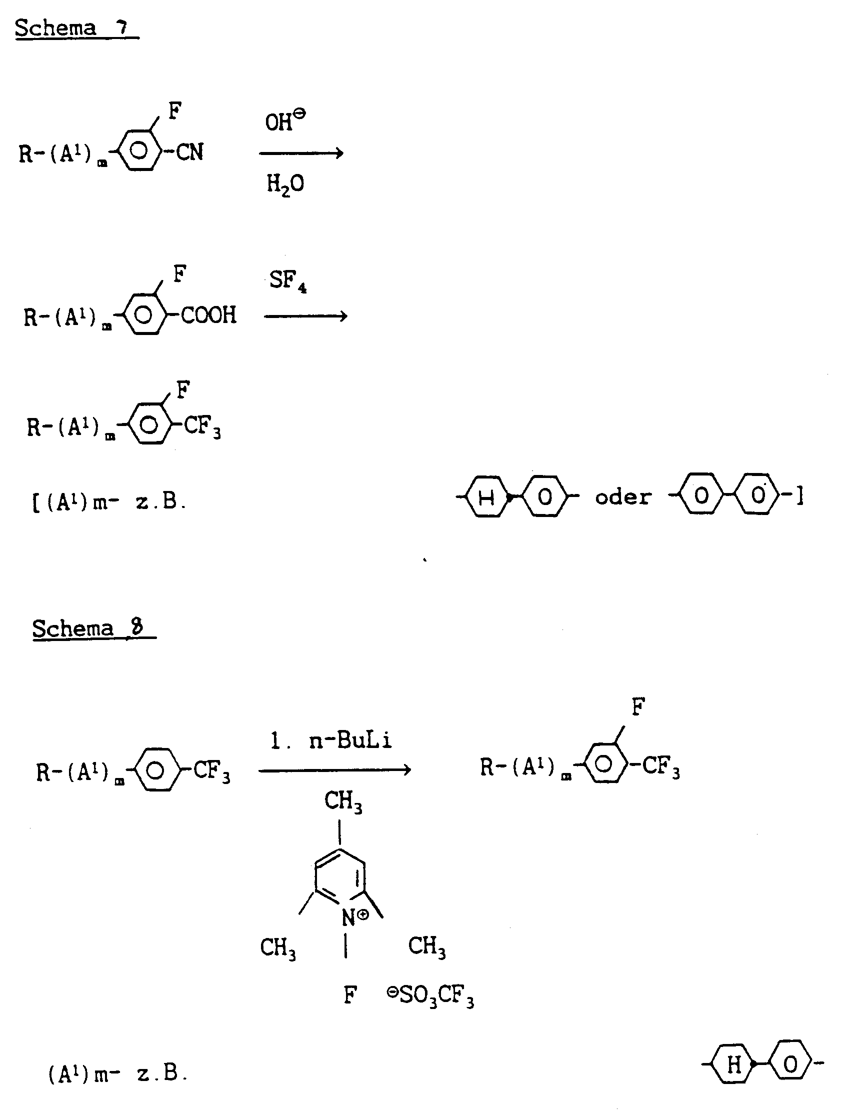

Ester der Formel I können auch durch Veresterung entsprechender Carbonsäuren (oder ihrer raktionsfähigen Derivate) mit Alkoholen bzw. Phenolen (oder ihren reaktionsfähigen Derivaten) oder nach der DCC-Methode (DCC = Dicyclohexylcarbodiimid) erhalten werden.

Die entsprechenden Carbonsäuren und Alkohole bzw. Phenole sind bekannt oder können in Analogie zu bekannten Verfahren hergestellt werden.

In einem weiteren Verfahren zur Herstellung der Verbindungen der Formel I setzt man ein Arylhalogenid mit einem Olefin um in Gegenwart eines tertiären Amins und eines Palladiumkatalysators (vgl. R.F. Heck, Acc. Chem. Res. 12 (1979) 146). Geeignete Arylhalogenide sind beispielsweise Chloride, Bromide und Iodide, insbesondere Bromide und Iodide. Die für das Gelingen der Kupplungsreaktion erforderlichen tertiären Amine, wie z.B. Triethylamin, eignen sich auch als Lösungsmittel. Als Palladiumkatalysatoren sind beispielsweise dessen Salze, insbesondere (Pd(II)-acetat, mit organischen Phosphor(III)-Verbindungen wie z.B. Triarylphosphanen geeignet. Man kann dabei in Gegenwart oder Abwesenheit eines inerten Lösungsmittels bei Temperaturen zwischen etwa 0° und 150°, vorzugsweise zwischen 20° und 100°, arbeiten; als Lösungsmittel kommen z.B. Nitrile wie Acetonitrile oder Kohlenwasserstoffe wie Benzol oder Toluol in Betracht. Die als Ausgangsstoffe eingesetzten Arylhalogenide und Olefine sind vielfach im Handel erhältlich oder können nach literaturbekannten Verfahren hergestellt werden, beispielsweise durch Halogenierung entsprechender Stammverbindungen bzw. durch Eliminierungsreaktionen an entsprechenden Alkoholen oder Halogeniden.

Auf diese Weise sind beispielsweise Stilbenderivate herstellbar. Die Stilbene können weiterhin hergestellt werden durch Umsetzung eines 4-substituierten Benzaldehyds mit einem entsprechenden Phoshorylid nach Wittig. Man kann aber auch Tolane der Formel I herstellen, indem man anstelle des Olefins monosubstituiertes Acetylen einsetzt (Synthesis 627 (1980) oder Tetrahedron Lett. 27, 1171 (1986)).

Weiterhin können zur Kopplung von Aromaten Arylhalogenide mit Arylzinnverbindungen umgesetzt werden. Bevorzugt werden diese Reaktionen unter Zusatz eines Katalysators wie z.B. eines Palladium(O)komplexes in inerten Lösungsmitteln wie Kohlenwasserstoffen bei hohen Temperaturen, z.B. in siedendem Xylol, unter Schutzgas durchgeführt.

Kopplungen von Alkinyl-Verbindungen mit Arylhalogeniden können analog dem von A.O. King, E. Negishi, F.J. Villani und A. Silveira in J. Org. Chem. 43, 358 (1978) beschriebenen Verfahren durchgeführt werden.

Tolane der Formel I können auch über die Fritsch-Buttenberg-Wiechell-Umlagerung (Ann. 279, 319, 1984) hergestellt werden, bei der 1,1-Diaryl-2-halogenethylene umgelagert werden zu Diarylacetylenen in Gegenwart starker Basen.

Tolane der Formel I können auch hergestellt werden, indem man die entsprechenden Stilbene bromiert und anschließend einer Dehydrohalogenierung unterwirft. Dabei kann man an sich bekannte, hier nicht näher erwähnte Varianten dieser Umsetzung anwenden.

Ether der Formel I sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, NaNH₂, NaOH, KOH, Na₂CO₃ oder K₂CO₃ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie z. B. Aceton, 1,2-Dimethoxyethan, DMF oder Dimethylsulfoxid oder auch mit einem Überschuß an wäßriger oder wäßrig-alkoholischer NaOH oder KOH bei Temperaturen zwischen etwa 20° und 100°.

Die Ausgangsmaterialien sind entweder bekannt oder können in Analogie zu bekannten Verbindungen hergestellt werden.

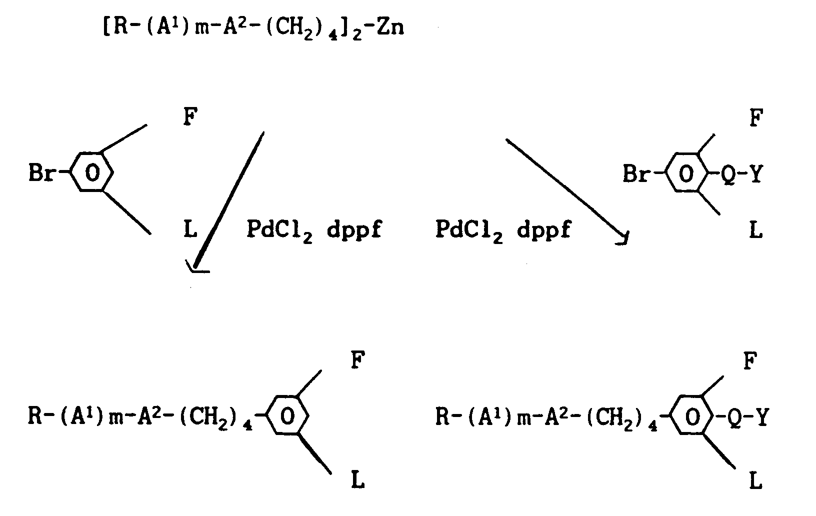

Bei der Pd(II)-katalysierten Kopplungsreaktion wird entweder direkt das Zielprodukt I' gebildet oder ein Vorprodukt, in das völlig analog zu den vorstehenden Methoden für Verbindungen oder Formel I der Rest -Q-Y eingeführt wird.

Die Verbindungen der Formel I' mit Z² = -CH=CH-CH₂CH₂- können noch Wittig gemäß folgendem Schema hergestellt werden:

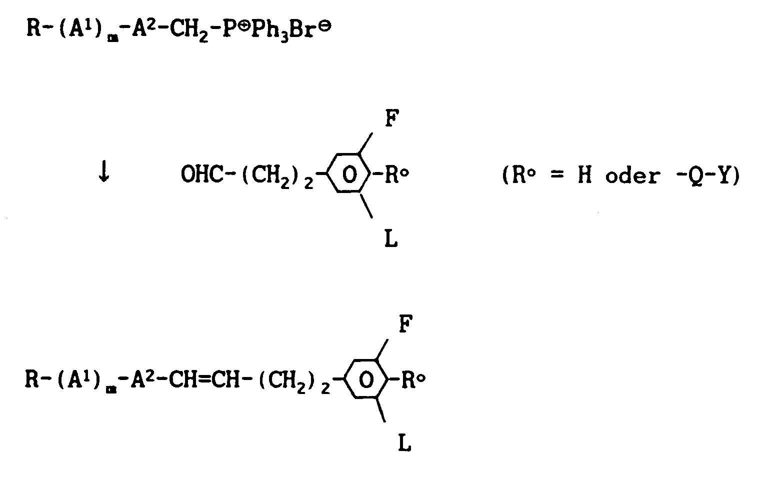

Die bevorzugten trans-Isomeren können nach können nach den literaturbekannten lsomerisierungsmethoden hergestellt werden. Die ggf. erhaltenen Vorprodukte mit R^{o} = H werden völlig analog zu den Vorprodukten der Verbindungen der Formel I durch Einführen des Restes -Q-Y in die Verbindungen der Formel I' übergeführt.

Die Aldehyde können durch Heck-Reaktion von entsprechend substituierten 1-Brom-3-fluorbenzolderivaten mit Allylalkohol erhalten werden.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäure-phenyl oder cyclohexyl-ester, Phenyl- oder Cyclohexyl-ester der Cyclohexylbenzoesäure, Phenyl- oder Cyclohexyl-ester der Cyclohexylcyclohexancarbonsäure, Cyclohexyl-phenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbidnungen lassen sich durch die Formeln 1, 2, 3, 4 und 5 charakterisieren:

R'-L-E-R'' 1

R'-L-COO-E-R'' 2

R'-L-OOC-E-R'' 3

R'-L-CH₂CH₂-E-R'' 4

R'-L-C≡C-E-R'' 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexylen, Pyr Pyrimidin-2-5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Rest L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R'' bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R'' voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R'' -F, -Cl, -NCS oder -(O)ᵢ CH₃₋₍ₖ₊₁₎ FₖCl₁, wobei i 0 oder 1 und k+1 1, 2 oder 3 sind; die Verbindungen, in denen R'' diese Bedeutung hat, werden mit den Teilformeln 1b, 2b, 3b, 4b und 5b bezeichnet. Besonders bevorzugt sind solche Verbindungen der Teilformeln 1b, 2b, 3b, 4b und 5b, in denen R'' die Bedeutung -F, -Cl, -NCS, -CF₃, OCHF₂ oder -OCF₃ hat.

In den Verbindungen der Teilformeln 1b, 2b, 3b, 4b und 5b hat R' die bei den Verbindungen der Teilformeln 1a-5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R'' -CN; diese Untergruppe wird im folgenden als Gruppe C bezeichnet und die Verbindungen dieser Untergruppe werden entsprechend mit Teilformeln 1c, 2c, 3c, 4c und 5 c beschrieben. In den Verbindungen der Teilformeln 1c, 2c, 3c, 4c und 5c hat R' die bei den Verbindungen der Teilformeln 1a-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbidnungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise
- Gruppe A:: 0 bis 90 %, vorzugsweise 20 bis 90 %, insbesondere 30 bis 90 %
- Gruppe B:: 0 bis 80 %, vorzugsweise 10 bis 80 %, insbesondere 10 bis 65 %
- Gruppe C:: 0 bis 80 %, vorzugsweise 5 bis 80 %, insbesondere 5 bis 50 %
wobei die Summe der Massenanteile der in den jeweiligen erfindungsgemäßen Medien enthaltenen Verbindungen aus den Gruppen A und/oder B und/oder C vorzugsweise 5 %-90 % und insbesondere 10 % bis 90 % beträgt.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroiti sche Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. Vor- und nachstehend bedeuten Potenzangaben Gewichtsprozent. Alle Temperaturen sind in Grad Celsius angegeben. Fp. bedeutet Schmelzpunkt Kp = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Δn bedeutet optische Anisotropie (589 nm, 20 °C) und die Viskosität (mm²/sec) wurde bei 20 °C bestimmt. In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Rest CₙH₂ₙ₊₁ und CₘH₂ₘ₊₁ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R¹, R², L¹ und L²:

| Code für R¹, R², L¹, L² | R¹ | R² | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| nT | CₙH₂ₙ₊₁ | CN | H | H |
| nN.F | CₙH₂ₙ₊₁ | CN | H | F |
| nF | CₙH₂ₙ₊₁ | F | H | H |
| nOF | OCₙH₂ₙ₊₁ | F | H | H |
| nCl | CₙH₂ₙ₊₁ | Cl | H | H |
| nF.F | CₙH₂ₙ₊₁ | F | H | F |
| nOmFF | CₙH₂ₙ₊₁ | OCₘH₂ₘ₊₁ | F | F |
| nmF | CₙH₂ₙ₊₁ | CₘH₂ₘ₊₁ | F | H |
| nCF₃ | CₙH₂ₙ₊₁ | CF₃ | H | H |
| nOCF₃ | CₙH₂ₙ₊₁ | OCF₃ | H | H |
| nOCF₂ | CₙH₂ₙ₊₁ | OCHF₂ | H | H |
| nS | CₙH₂ₙ₊₁ | NCS | H | H |
| rVsN | CᵣH₂ᵣ₊₁-CH=CH-CₛH₂ₛ- | CN | H | H |
| rEsN | CᵣH₂ᵣ₊₁-O-CₛH₂ₛ- | CN | H | H |
| nNF | CₙH₂ₙ₊₁ | CN | F | H |
| nAm | CₙH₂ₙ₊₁ | COOCₘH₂ₘ₊₁ | H | H |

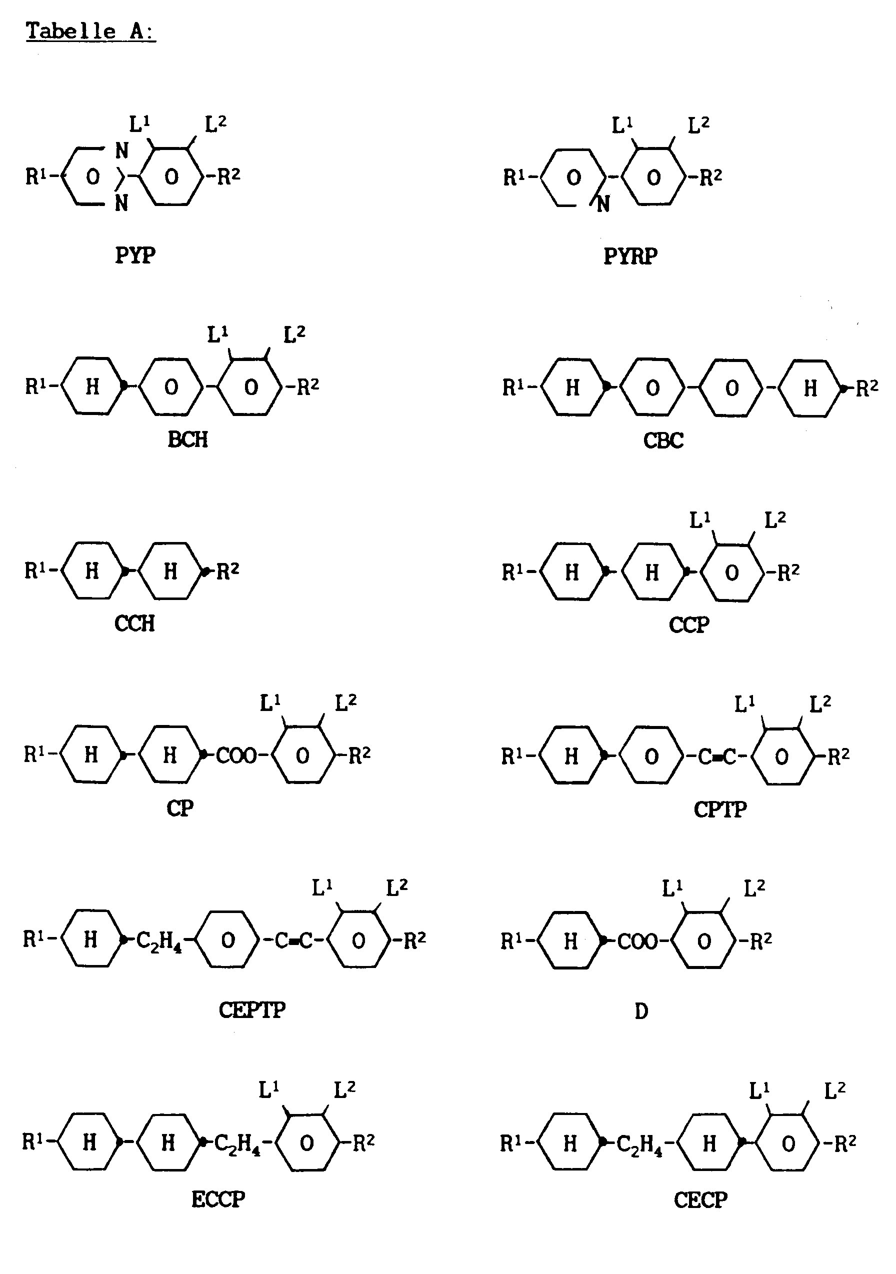

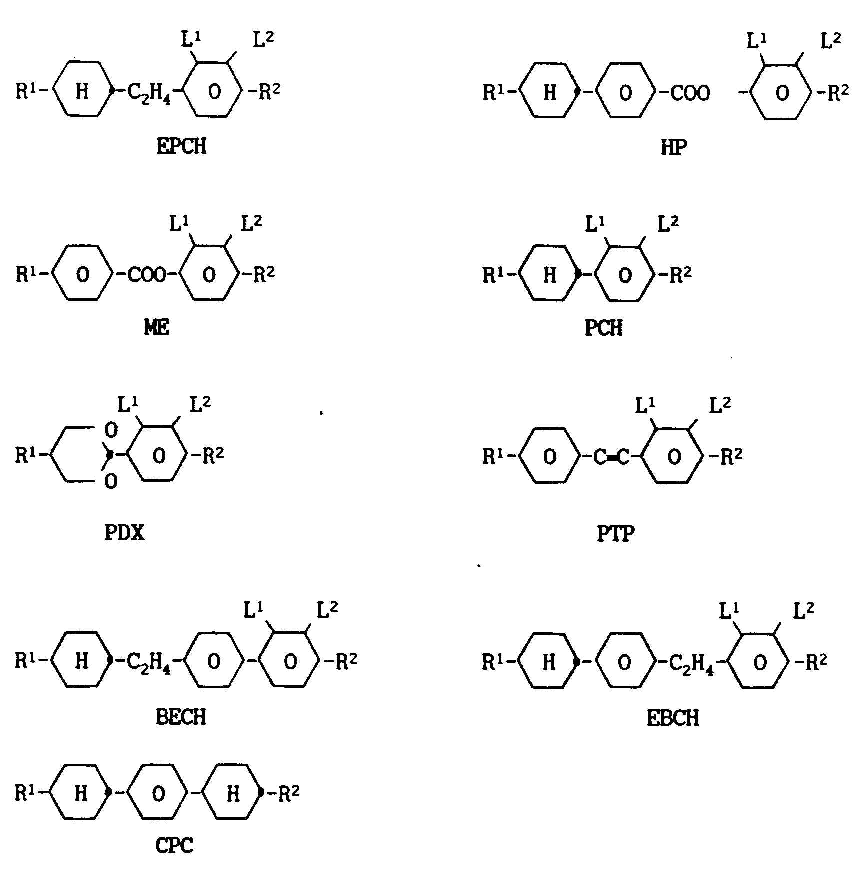

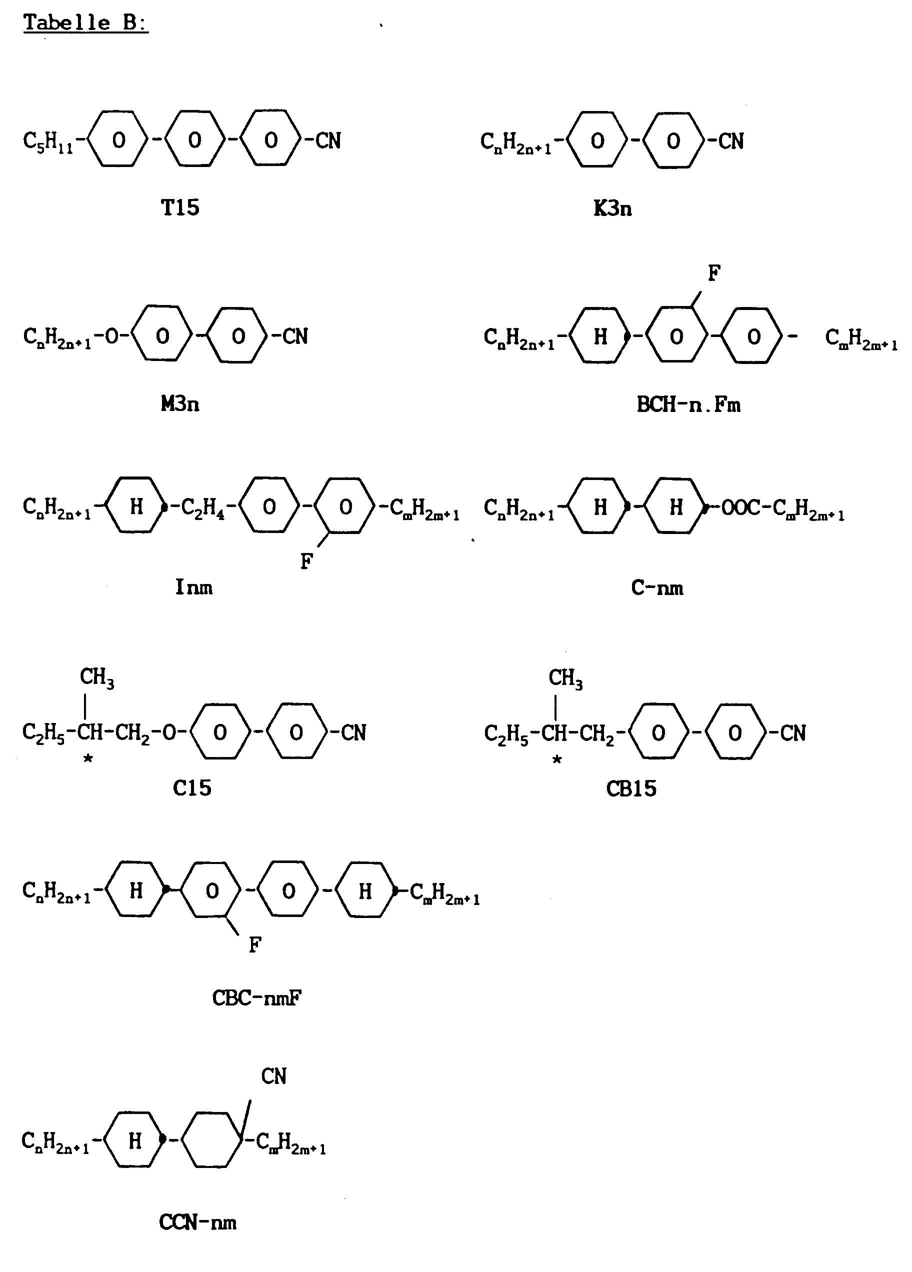

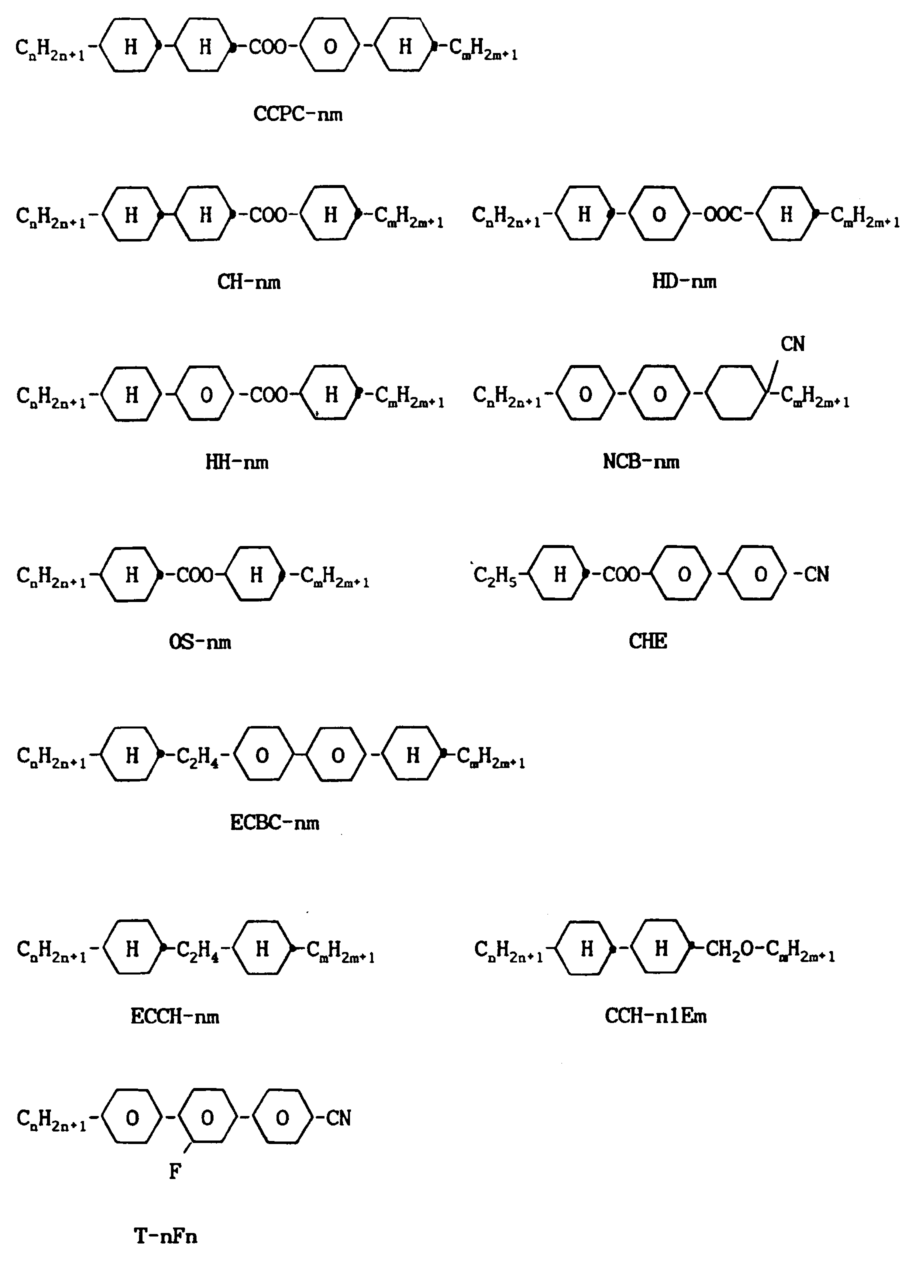

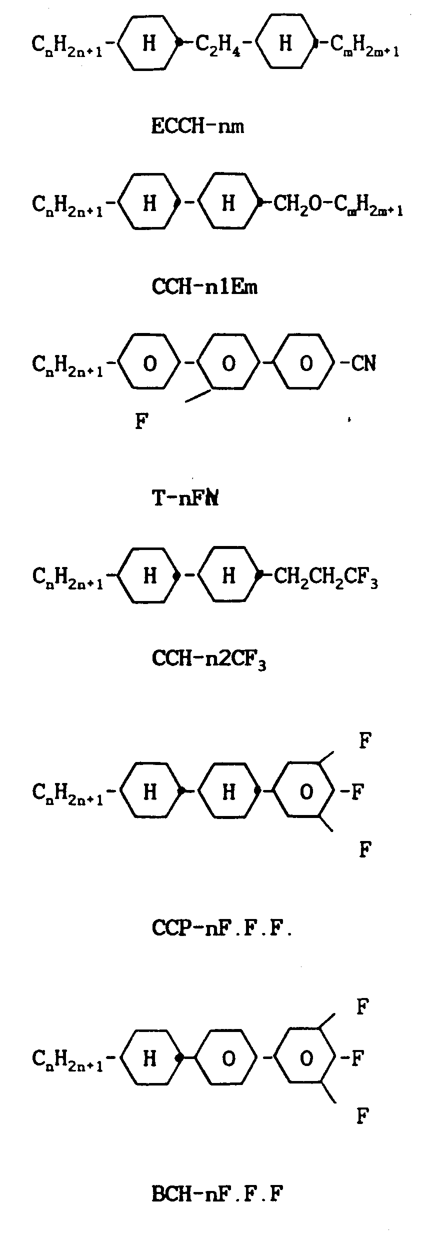

"Übliche Aufarbeitung" bedeutet: man gibt gegebenenfalls Wasser hinzu, extrahiert mit Methylenchlorid, Diethylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:
- DAST: Diethylaminoschwefeltrifluorid
- DCC: Dicyclohexylcarbodiimid
- DDQ: Dichlordicyanobenzochinon
- DIBALH: Diisobutylaluminiumhydrid
- KOT: Kalium-tertiär-butanolat
- THF: Tetrahydrofuran
- pTSOH: p-Toluolsulfonsäure
- TMEDA: Tetramethylethylendiamin

Aus diesem Phenol erhält man OCHF₂-Derivat durch Einleiten von CHClF₂ in die THF-Lösung des Phenolats. Nach extraktiver Aufarbeitung und üblicher Reinigung erhält man 1-[trans-4-(trans-4-n-Propylcyclohexyl)-cyclohexyl]-2-(4-difluormethoxy-3,5-difluorphenyl)-ethan.

### Beispiel 2 bis 23:

Analog erhält man aus den entsprechenden Vorstufen der Formel II die folgenden erfindungsgemäßen Verbindungen:

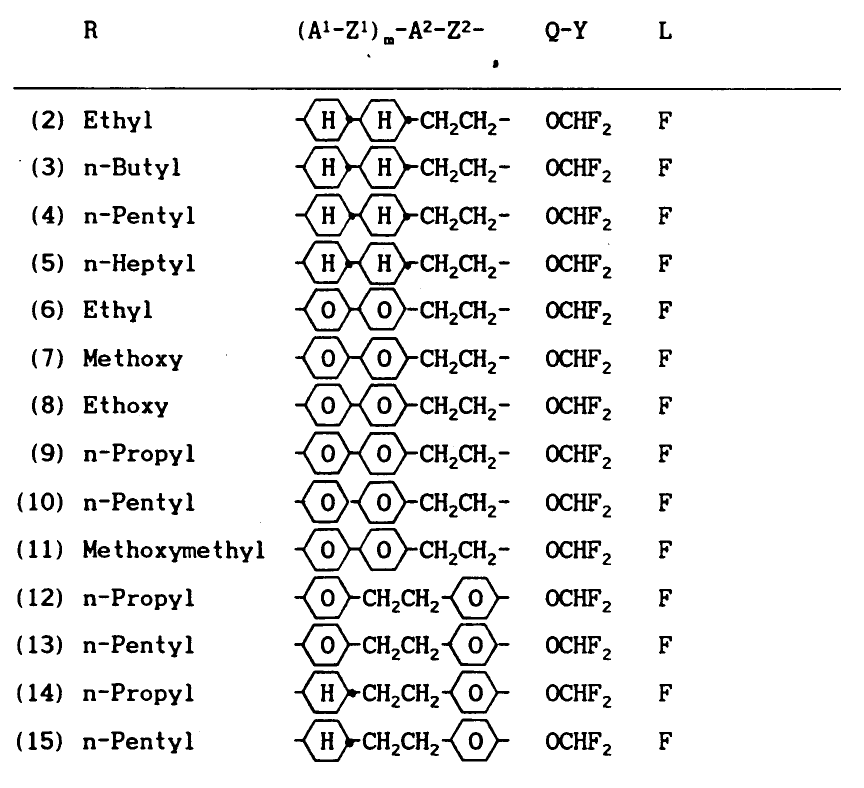

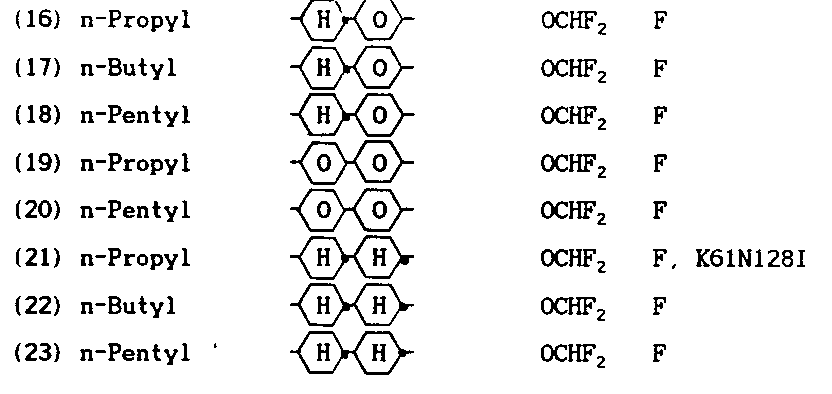

### Beispiel 24

In einem Autoklaven, der auf 0° gekühlt ist, füllt man 2 mol wasserfreie Flußsäure. Dann gibt man eine Mischung aus 0,18 mol Tetrachlormethan und 0,06 mol 1-[trans-4-(trans-4-n-Propylhexyl)-cyclohexyl]-2-(4-hydroxy-3,5-difluorphenyl)-ethan (Beispiel 1) zu. Die Mischung wird ca. 8 Stunden bei 150° gerührt, abgekühlt, auf Eiswasser gegossen und mit Ether nachgewaschen. Die beiden Phasen werden ca. 30 Minuten gerührt, getrennt und die Etherlösung mit 5%iger KOH zur alkalischen Reaktion gewaschen. Nach Trocknen, Abfiltrieren, Abdestillieren und Reinigung erhält man 1-[trans-4-(trans-4-n-Propylcyclohexyl)-cyclohexyl]-2-(4-trifluormethoxy-3,5-difluorphenyl)-ethan.

### Beispiel 25 bis 45:

Analog erhält man aus den entsprechenden Vorstufen der Formel II die folgenden Verbindungen:

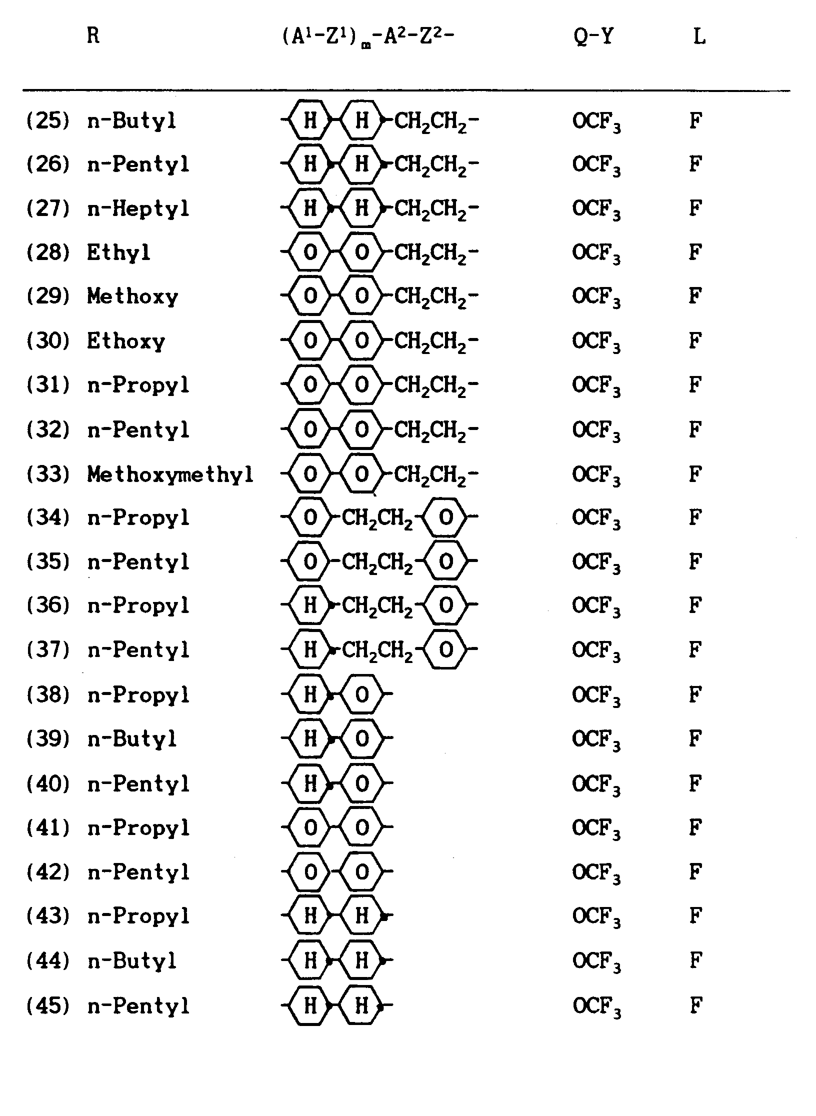

### Beispiel 46

In einem Hastelloy-Autoklaven werden 31,6 g 2-Fluor-4-[trans-4-(trans-4-n-propylcyclohexyl)-cyclohexyl)-phenol (hergestellt aus dem entsprechenden Benzylether durch Hydrierung, der durch Kreuzkopplung von Bis-[trans-4-(trans-4-n-propylcyclohexyl)-cyclohexyl]-zink mit 4-Brom-2-fluorbenzyloxybenzol erhalten wurde) und 49,3 g Tetrachlorkohlenstoff vorgelegt und mit Trockeneis/Aceton gekühlt.

Nach Anlegen eines Vakuums wird mit 66,7 g HF und 1,67 g BF₃ versetzt. Nach achtstündigem Rühren bei 150 °C läßt man auf Zimmertemperatur abkühlen, zieht das HF durch einen Aspirator ab. Der Rückstand wird in Methylenchlorid aufgenommen und mit NaF versetzt um restliches HF abzutrennen. Nach dem Filtrieren wird der Eindampfrückstand durch Chromatographie über Kieselgel und mehrfache Kristallisation aus Hexan und Ethanol aufgereinigt. Man erhält 2-Fluor-4-[trans-4-(trans-4-n-propylcyclohexyl)-cyclohexyl)-trifluormethoxybenzol.

### Beispiel 47 bis 68:

Analog erhält man aus den entsprechenden Phenolen die folgenden Verbindungen:

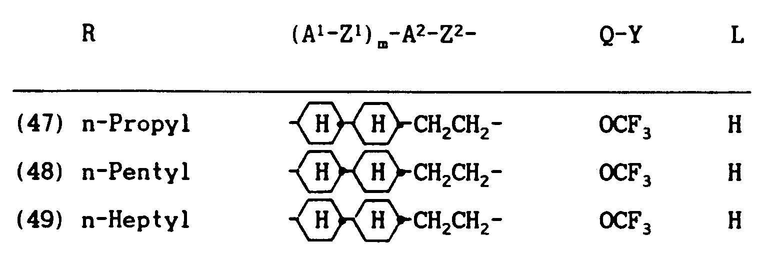

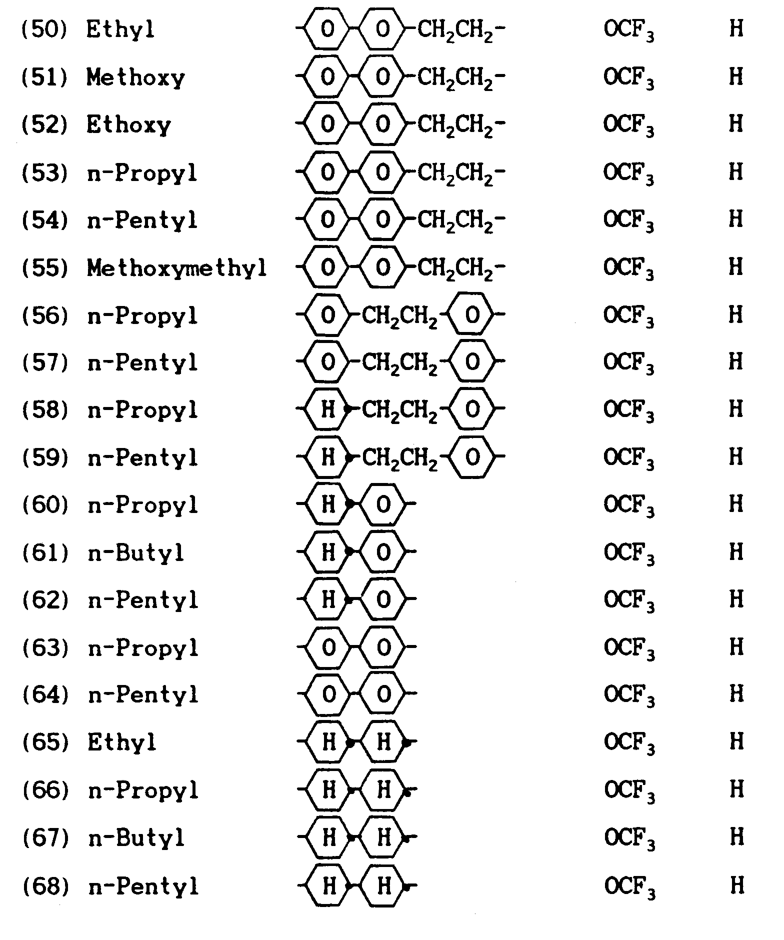

### Beispiel 69 bis 91:

Analog Beispiel 1 erhält man aus den entsprechenden Phenolen die folgenden Verbindungen:

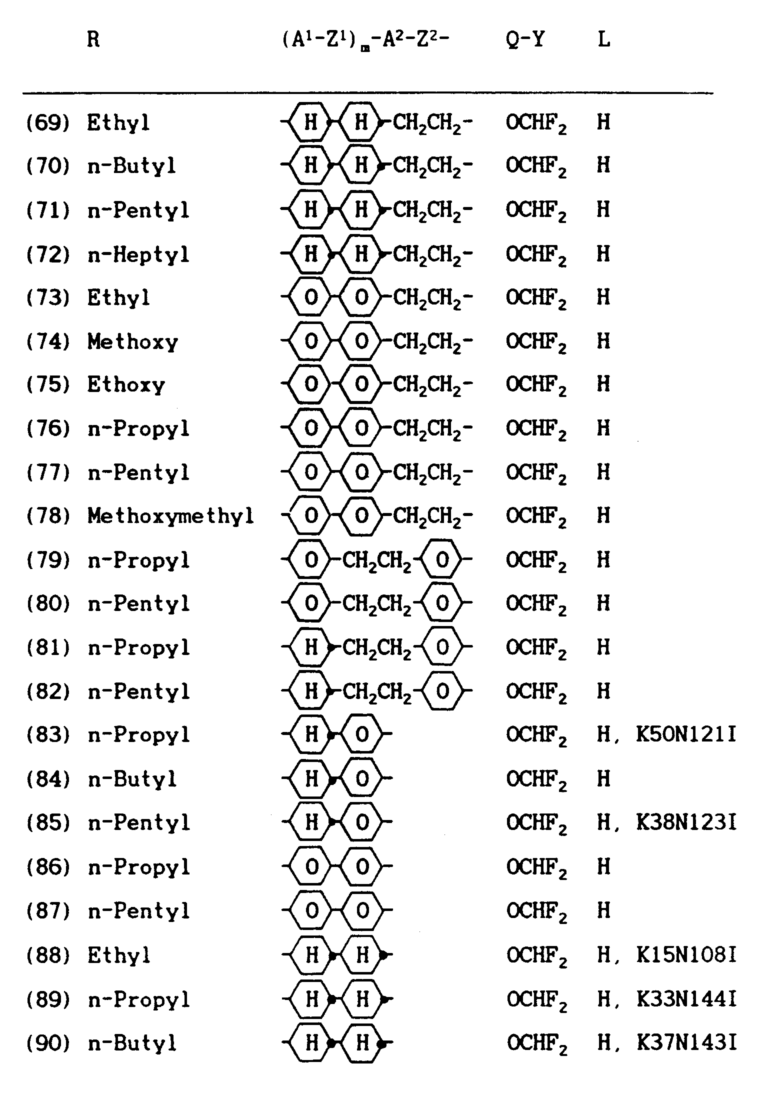

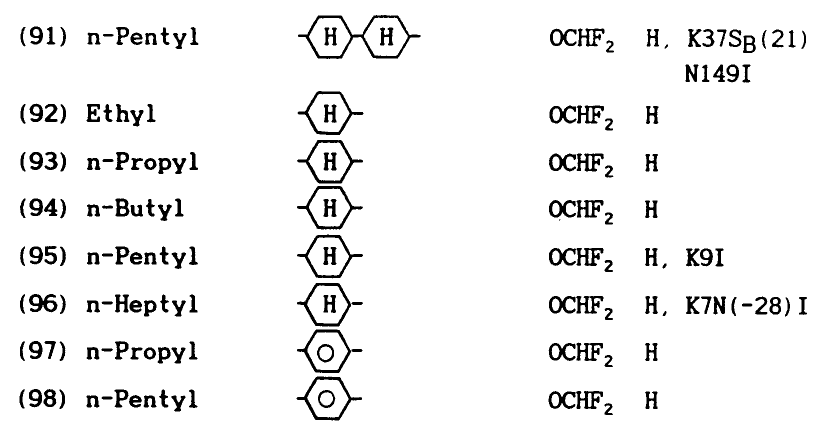

### Beispiel 99

Eine Lösung von 0,1 m 1-[trans-4-(trans-4-n-Propylcyclohexyl)-cyclohexyl]-2-(3,5-difluorphenyl)-ethan (hergestellt nach Schema 3) und 0,1 m TMEDA in 300 ml THF wird bei ca. -65° mit 0,1 m n-BuLi (1,5 M in Hexan) tropfenweise versetzt. Man rührt noch 30 Min. bei dieser Temperatur und setzt dann 0,2 m N-Chlorsuccinimid in 70 ml THF langsam zu. Nach beendeter Zugabe läßt man auf -20° erwärmen und hydrolysiert mit H₂O. Durch Zugabe von Diethylether wird das Produkt vollständig in Lösung gebracht. Nach extraktiver Aufarbeitung und Reinigung durch Chromatographie und Kristallisation erhält man 1-[trans-4-(trans-4-n-Propylcyclohexyl)-cyclohexyl]-2-(4-chlor-3,5-difluorphenyl)-ethan, K 88 N 129 I

### Beispiel 100 bis 143

Analog erhält man aus den entsprechenden Vorstufen der Formel II die folgenden erfindungsgemäßen Verbindungen:

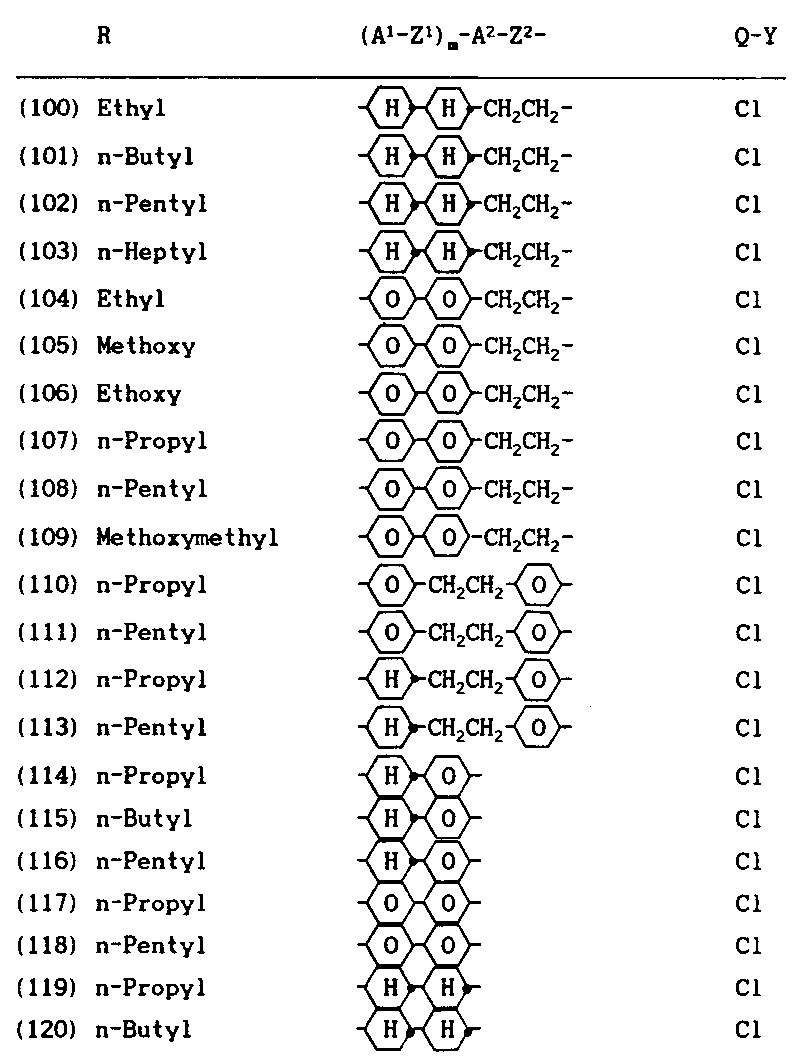

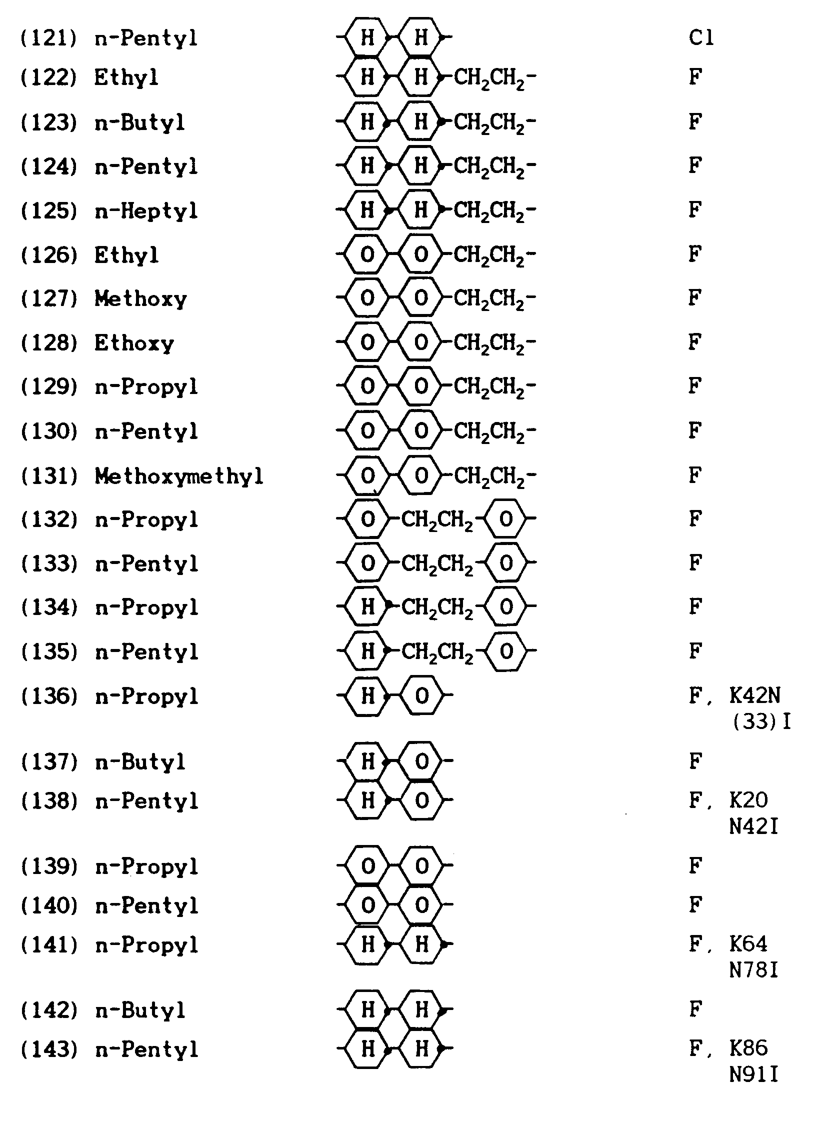

### Beispiel 144

Ein Gemisch von 0,1 m 4-(trans-4-n-Pentylcyclohexyl)-2-fluorbenzoesäure und 0,3 m SF₄ wird im Autoklaven 8 Stunden auf 130° erhitzt. Das erhaltene Rohprodukt wird in Pentan aufgenommen und extraktiv aufgearbeitet. Nach üblicher Aufreinigung durch Destillation und Kristallisation erhält man 4-(trans-4-n-Pentylcyclohexyl)-2-fluortrifluortoluol.

### Beispiel 145

Eine Lösung von 0,01 m 4-(trans-4-n-Propylcyclohexyl)-4'-trifluormethylbiphenyl in 10 ml n-Hexan wird mit 0,01 m TMEDA versetzt. Bei 0-5 °C setzt man 0,01 m n-BuLi zu und rührt noch 1/2 h bei RT und 1/2 h bei 40 °C nach. Dann kühlt man auf 0 °C ab, setzt 20 ml THF zu und tropft bei dieser Temperatur gemäß Schema 21 das Fluorierungsmittel 1-Fluor-2,4,6-trimethylpyridinium Triflat (in THF) zu. Nach extraktiver Aufarbeitung erhält man wie üblich durch Chromatographie und Kristallisation 4-(trans-4-n-Propylcyclohexyl)-3'-fluor-4'-trifluormethylbiphenyl.

### Beispiel 146 bis 175:

Analog Beispiel 144 bzw. 145 erhält man aus den entsprechenden Vorprodukten die folgenden Verbindungen:

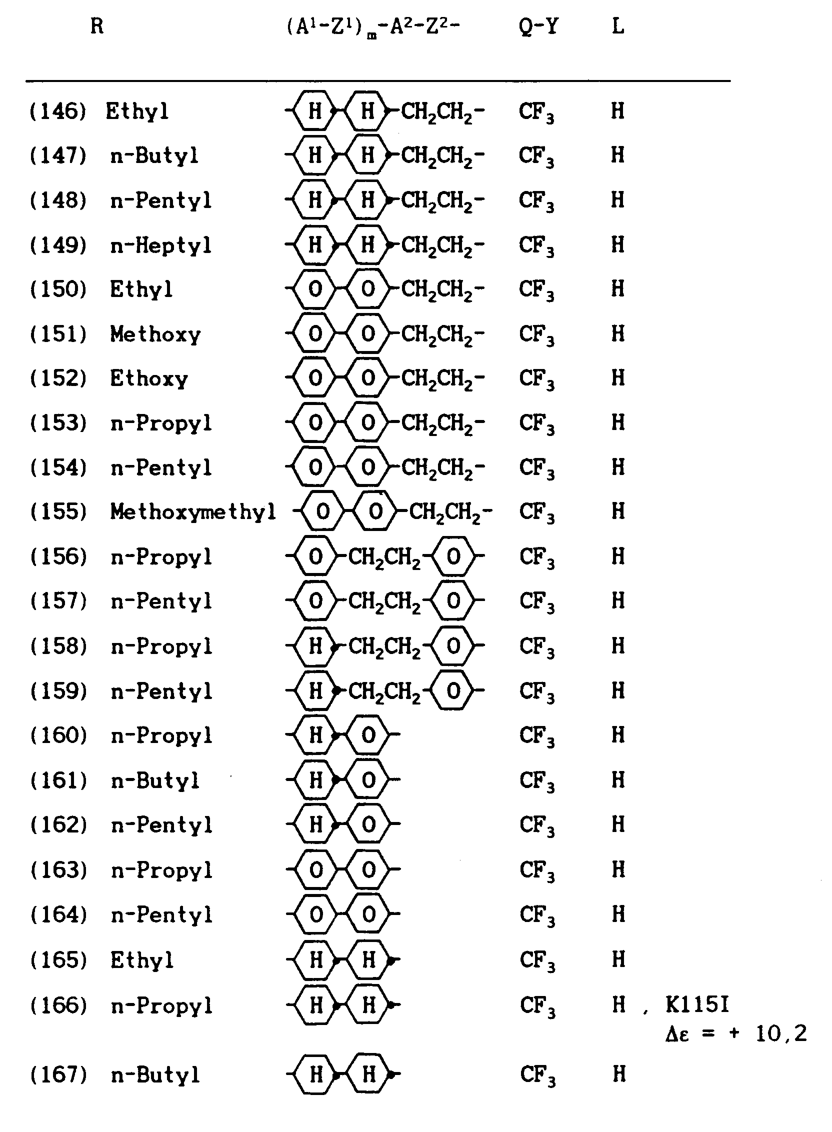

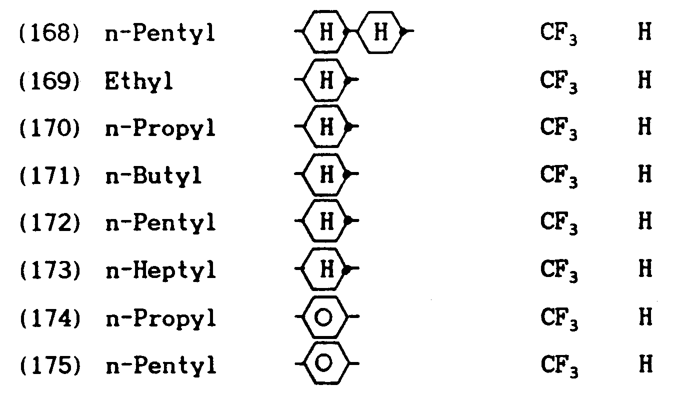

### Beispiel 176

Ein Gemisch von 0,025 m CuJ, 0,0125 m CF₃COONa und 0,0125 m 1-[trans-4-(trans-4-n-Propylcyclohexyl)-cyclohexyl]-2-(4-jod-3,5-difluorphenyl)-ethan [erhältlich gemäß Schema 15] wird in 100 ml N-Methylpyrrolidon unter Rühren auf 150° erhitzt. Nach einer Stunde arbeitet man wie üblich extraktiv auf und erhält nach chromatographischer Aufreinigung 1-[trans-4-(trans-4-n-Propylcyclohexyl)-cyclohexyl]-2-(4-trifluormethyl-3,5-difluorphenyl)-ethan.

### Beispiel 177 bis 206:

Analog Beispiell 176 erhält man aus den entsprechenden 3,5-Difluorphenyl-Verbindungen die folgenden Verbindungen:

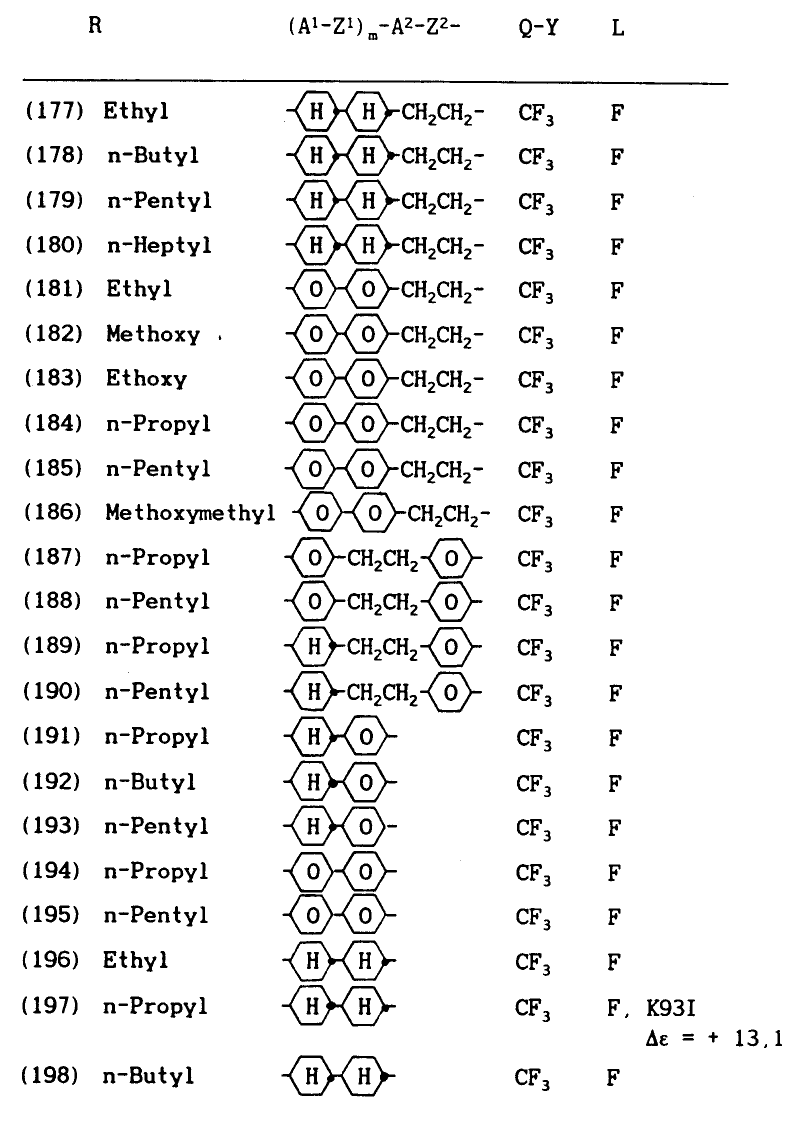

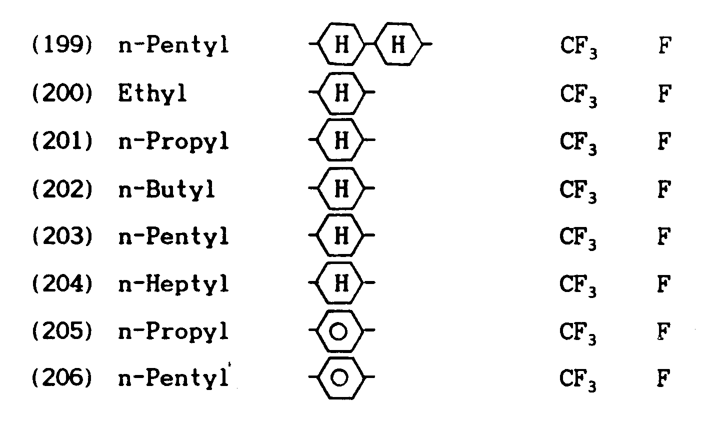

### Beispiel 207

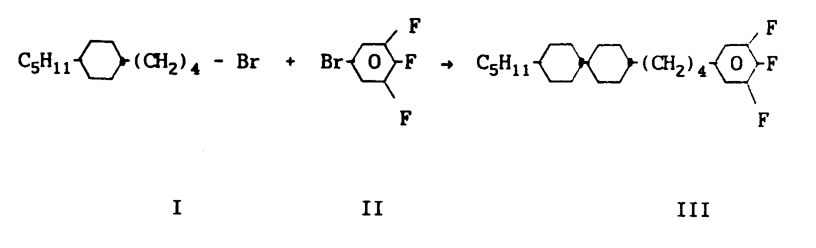

37,1 g I (0,1 Mol) werden in 150 ml eines Lösungsmittelgemisches von THF/Toluol (1 : 4 - Volumenverhältnis) vorgelegt, dann 11,5 g Zinkbromid wasserfrei und darauf 1,4 g Lithiumgranulat hinzugefügt. Das Gemisch wird unter Argon und Rühren 4 Std. zwischen 0 °C und 10 °C mit Ultraschall behandelt, um I in die entsprechende Dialkylzinkverbindung zu überführen. Die zinkorganische Verbindung wird mit 21,1 g II (0,1 Mol) und 1,5 g (2 mol %) 1,1'-Bis(diphenylphosphino)-ferrocen-palladium(II)dichlorid (PdCl₂ dppf) versetzt und nach Entfernung des Ultraschallbades und der Kühlung 24 h bei Zimmertemperatur gerührt. Es wird mit 100 ml gesättigter NH₄Cl-Lösung unter Rühren zersetzt, die organische Phase abgetrennt, die wäßrige Phase 2 x mit Toluol extrahiert. Die vereinigten organischen Extrakte liefern nach dem Trocknen, Einengen und Chromatografieren über Kieselgel mit Hexan III. (I ist herstellbar durch Kettenverlängerung von 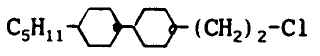 mittels Malonester.) Mit II lassen sich analog I die nachfolgend aufgeführten Alkylbromide umsetzen:

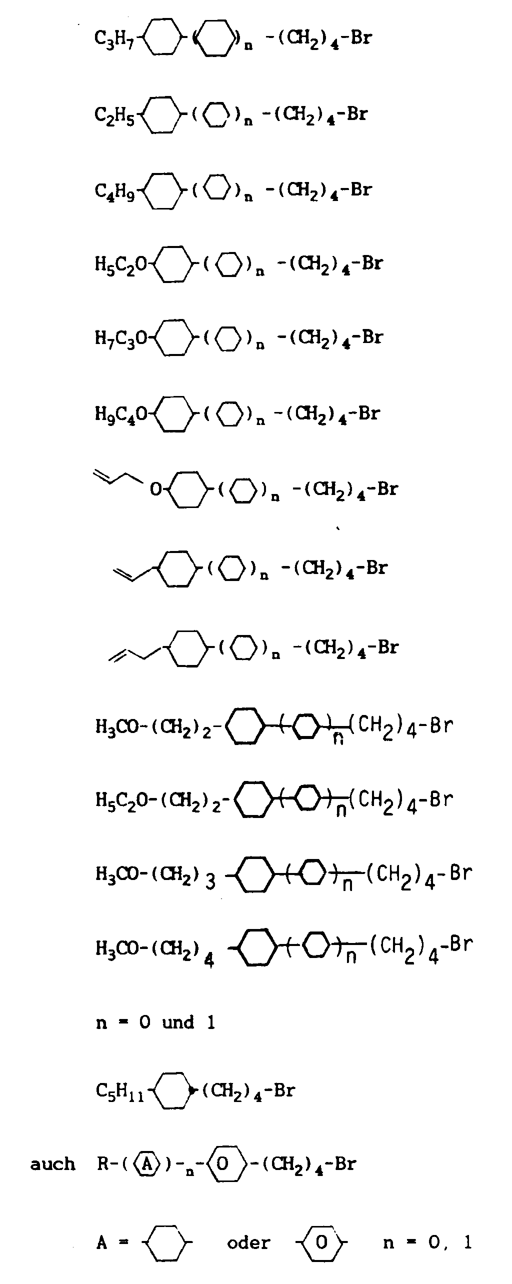

### Beispiel 208

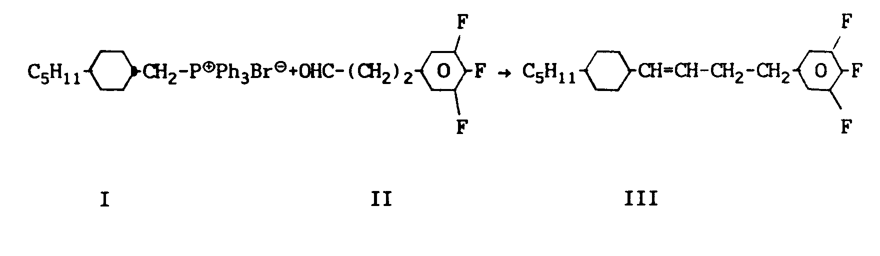

50,9 g (0,1 Mol) Wittigsalz I und 17,2 g Aldehyd II (hergestellt durch Heck-Reaktion von 1-Bromtrifluorbenzol mit Allylalkohol) werden mit 11,5 g Kalium-tert-butylat zwischen 0 °C und 10 °C portionsweise versetzt. Nach der Zugabe wird 24 Std. bei Zimmertemperatur gerührt, auf Wasser gegossen, neutralisiert, mit Toluol extrahiert und der Toluolextrakt nach dem Trocknen eingedampft und über Kieselgel mit Hexan filtriert. Es werden 28 g III erhalten.

### Beispiel 209

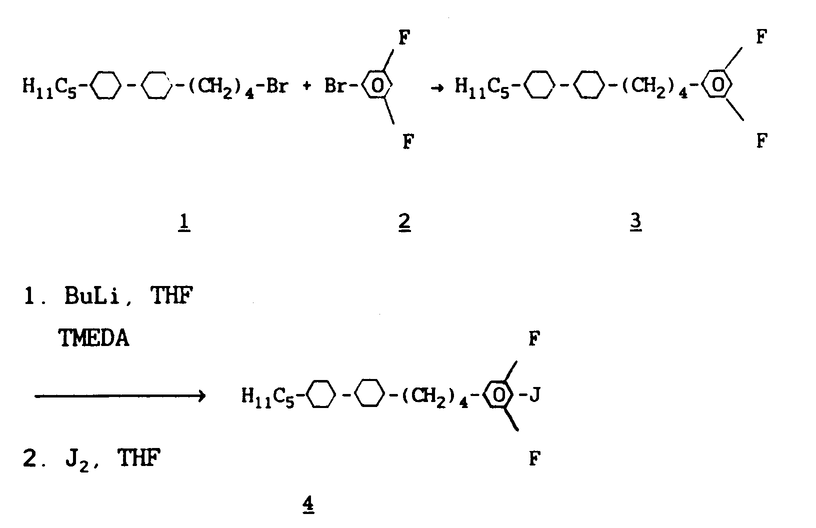

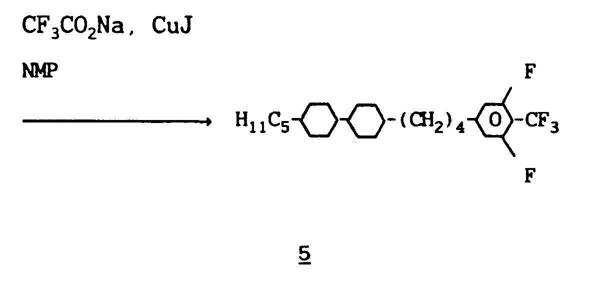

37,1 g (100 mmol) 1 werden analog zum Beispiel 207 durch Umsetzung mit 2 in 3 überführt.

Zu einem Gemisch aus 19,0 g (47 mmol) 3, 7,5 ml TMEDA (50 mmol) und 150 ml THF werden bei -65 bis -70 °C 31 ml n-Buli (15 % in Hexan) zugetropft und es wird 1 Stunde bei -70 °C nachgerührt. Dann wird bei -65 bis -70 °C eine Lösung von 12,0 g (47 mmol) Jod in 25 ml THF zugetropft und 0,5 h bei -70 °C nachgerührt. Man erwärmt auf -30 °C, hydrolysiert mit 15 ml Wasser und reduziert überschüssiges Jod durch Zusatz von 15 ml Natriumhydrogensulfitlösung. Nach üblicher Aufarbeitung und Umkristallisation aus Heran erhält man 23,9 g (45 mmol) 4. Aus einem Gemisch von 20,2 g (38 mmol) 4, 4,4 g (76 mmol) KF, 22,8 g (168 mmol) Natriumtrifluoracetat und 800 ml NMP werden bei 70 °C und 4 mbar 400 ml NMP abdestilliert. Dann gibt man 14,4 g (76 mmol) getrocknetes CuJ zum Reaktionsgemisch und rührt 5 h bei 160 °C. Anschließend werden ca. 300 ml NMP abdestilliert. Man läßt auf RT abkühlen und versetzt mit 300 ml MTB-Ether. Man wäscht mit Wasser, trocknet mit Na₂SO₄, filtriert und engt zum Rückstand ein. Nach Chromatographie an Kieselgel mit Hexan erhält man 5.

### Beispiel 210

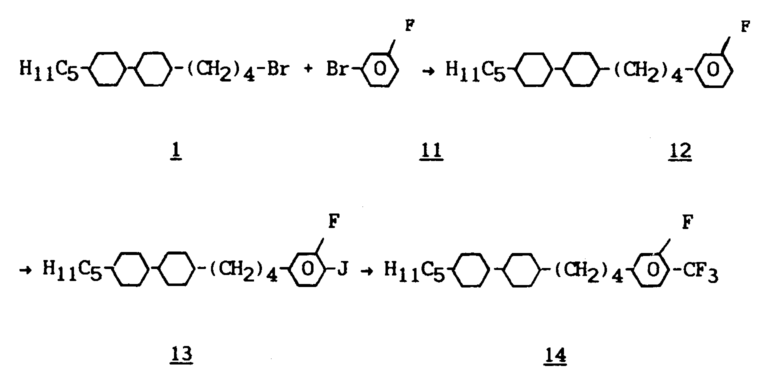

31,1 g (100 mmol) 1 werden analog zum Beispiel 207 durch Umsetzung mit 11 in 12 überführt.

Zu einem Gemisch aus 18,2 g (47 mmol) 12, 7,4 g Kaliumtertiärbutylat und 110 ml THF werden bei -100 °C 40 ml n-BuLi zugetropft und es wird 1 h bei -100 °C nachgerührt. Anschließend wird bei -85 bis -90 °C eine Lösung von 15,9 g Jod in 60 ml THF zugetropft. Man rührt noch 0,5 h bei -90 °C nach, erwärmt auf -30 °C, hydrolysiert mit 30 ml Wasser, säuert mit konz. Salzsäure an und reduziert überschüssiges Jod durch Zusatz von Natriumhydrogensulfitlösung. Nach üblicher Aufarbeitung und Umkristallisation aus Hexan erhält man 21,4 g (41 mmol) 13. 19,5 g (38 mmol) 13 werden entsprechend Beispiel 209 durch Umsatz mit Natriumtrifluoracetat in 14 überführt. Man erhält nach chromatographischer Aufreinigung 14.

### Beispiel 211

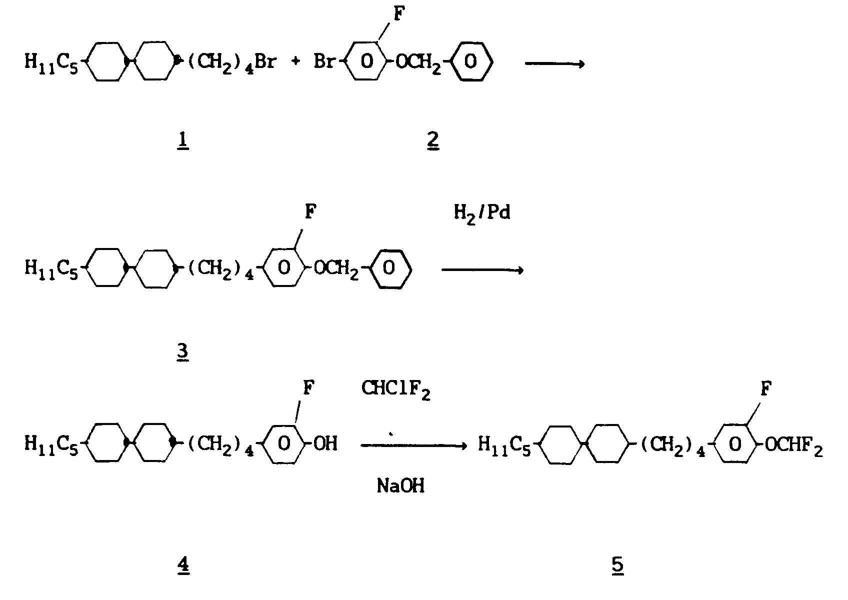

Gemäß obenstehendem Syntheseschema wird analog Beispiel 207 nach Überführung von 1 in die zinkorganische Verbindung durch eine Pd-(II)-katalysierte Kopplungsreaktion mit 2 die Verbindung 3 erhalten. Hydrogenolytische Spaltung des Benzylethers führt zum Phenol 4.

4,0 g (0,01 mol) dieses Phenols werden in THF vorgelegt, 3,1 g 32 %ige Natronlauge und 0,5 g Tetrabutylammoniumhydrogensulfat zugegeben, auf 50 °C erwärmt und unter Rühren solange Chlordifluormethan eingeleitet, bis dieses an einem mit Trockeneis gekühlten Kühler kondensiert. Nach dem Abkühlen wird die THF-Lösung von dem ausgefallenen schmierigen Produkt abdekantiert, eingeengt und das erhaltene 5 aus Ethanol umkristallisiert.

### Beispiel 212

Zu einer Lösung von 15 g 4-(trans-4-n-Propyl-cyclohexyl)-2-fluor-4'-brombiphenyl in 50 ml THF tropft man bei -70 °C 25 ml Butyllithium (15 % in Hexan) und gibt nach 30 min eine gekühlte Lösung von 4,5 g ZnBr₂ in 25 ml THF zu. Nach Zugabe von 8,5 g 1-Brom-3,4,5-trifluorbenzol in 75 ml THF und 0,6 g PdCl₂ - dppf läßt man auf Raumtemperatur kommen. Nach 24 h Rühren gießt man in 100 ml gesättigte NH₄Cl-Lösung und arbeitet wie üblich auf. Man erhält 4''-(trans-4-n-Propylcyclohexyl)-2'',3,4,5-tetrafluorterphenyl, K 148 N 233 I.

Analog erhält man aus den entsprechenden Bromaromaten:
4''-n-Propyl-2'',3,4,5-tetrafluorterphenyl
4''-n-Pentyl-2'',3,4,5-tetrafluorterphenyl
4'-(trans-4-methoxymethylcyclohexyl)-3,4,5-trifluorbiphenyl

### Beispiel 213

Ein Gemisch von 10,5 g 1-Brom-3,4,5-trifluorbenzol, 7,4 g p-Ethoxystyrol, 0,25 g Pd-acetat, 0,6 g Tri-o-tolylphosphin, 7 g Triethylamin und 125 ml Acetonitril wird am Rückfluß erhitzt bis zur Beendigung der Reaktion. Nach üblicher Aufarbeitung erhält man 1-(p-Ethoxyphenyl)-2-(3,4,5-trifluorphenyl)-ethen (K 76 I), welches an Pd-C (5 %) in THF zu 1-(p-Ethoxyphenyl)-2-(3,4,5-trifluorphenyl)-ethan hydriert wird, K 45 I.

### Beispiel 214

Aus p-Ethoxystyrol und dem Benzylether des p-Brom-o-fluorphenols erhält man analog Beispiel 213 1-(p-Ethoxyphenyl)-2-(3-fluor-4-benzyloxyphenyl)-ethen. Durch Hydrieren an Pd-C (5 %) im THF erhält man 1-(p-Ethoxyphenyl)-2-(4-hydroxy-3-fluorphenyl)-ethan, wovon 41,3 g zusammen mit 5,6 g Tetrabutylammoniumhydogensulfat bei 40 °C in 500 ml THF gelöst werden. In diese Lösung werden 43 g Chlordifluormethan so eingeleitet, daß eine geringe Menge am Trockeneisrückflußkühler kondensiert. Dann werden 32 g NaOH (50 %) unter gutem Rühren innerhalb von 10 min zugetropft. Nach einer weiteren Stunde Rühren bei 40° wird abgekühlt und von dem gebildeten schmierigen Niederschlag abdekantiert. Nach Abdampfen des Lösungsmittels wird der Rückstand chromatographisch und durch Umkristallisation aufgereinigt. Man erhält 1-(p-Ethoxyphenyl)-2-(3-fluor-4-difluormethoxyphenyl)-ethan, K 43 I.

### Beispiel 215

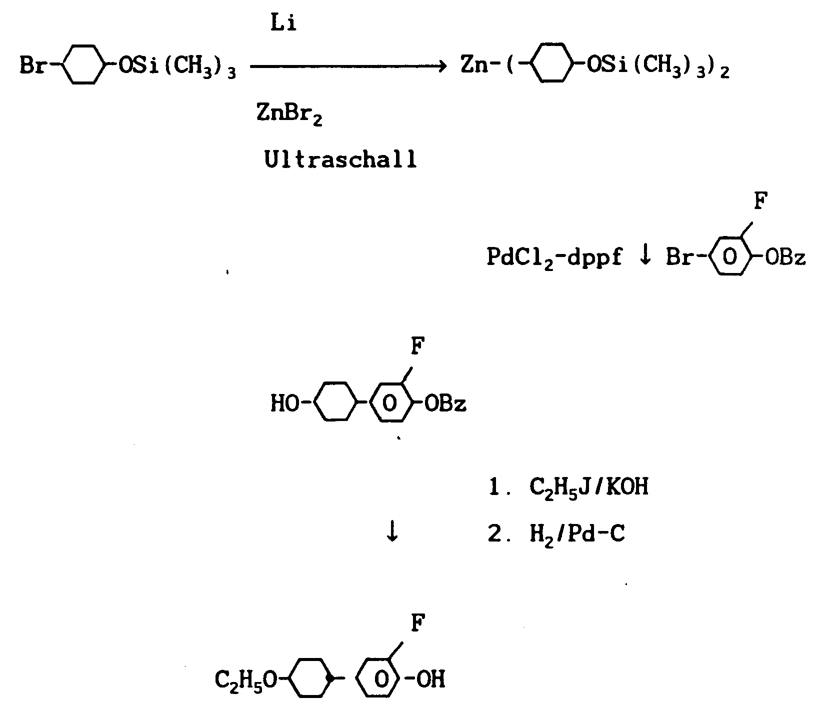

Zu einer Lösung von 8,2 des erhaltenen p-(trans-4-Ethoxycyclohexyl)-o-fluorphenol in 80 ml THF werden 11 g NaOH (32 %) zugegeben und 13,8 g Chlordifluormethan eingeleitet (Trockeneisrückflußkühler). Nach einer Stude nachrühren wird vom Niederschlag abdekantiert und die Lösung zum Rückstand eingeengt. Durch Kugelrohrdestillation erhält man p-(trans-4-Ethoxycyclohexyl)-o-fluordifluormethoxybenzol, Kp₁ ∼ 220 °C, Tg-62°.

### Beispiel 216

Aus 2-n-Octyloxy-5-(3-fluor-4-hydroxyphenyl)-pyridin [hergestellt durch Umsetzung von 2,5-Dibrompyridin mit NaH/1-Octanol, Überführung des 2-n-Octyloxy-5-brompyridin in die Zinkverbindung (BuLi/ZnBr₂/-70°), Kreuzkopplung mit 3-Fluor-4-acetoxybrombenzol (PdCl₂ dppf/THF) und Verseifung der Acetylgruppe mit KOH/MeOH] erhält man analog Beispiel 214 2-n-Octyloxy-5-(3-fluor-4-difluormethoxyphenyl)-pyridin, K 29 I.

Analog werden hergestellt:
2-n-Octyl-5-(3-fluor-4-difluormethoxy-phenyl)-pyridin, K 29 I.

| Beispiel A | | | |
|---|---|---|---|
| PCH-5F | 10,0 % | S → N [°C] | < -40 |
| PCH-6F | 7,0 % | Klärpunkt [°C] | +64 |
| PCH-7F | 15,0 % | Viskosität 20 °C | 14 |
| CCP-20CF₃ | 10,0 % | Δn (589 nm, 20 °C) | 0,0800 |
| CCP-30CF₃ | 12,0 % | n_{ε} (589 nm, 20 °C) | 1,5576 |
| CCP-40CF₃ | 8,0 % | V_{(10,0,20)} | 1,61 |
| CCP-50CF₃ | 12,0 % | V_{(50,0,20)} | 2,04 |
| CCP-3F.F.F | 10,0 % | V_{(90,0,20)} | 2,66 |
| BCH-5F.F | 16,0 % | | |

| Beispiel B | | | |
|---|---|---|---|
| PCH-5F | 12,0 % | S → N [°C] | < -30 |
| PCH-7F | 8,0 % | Klärpunkt [°C] | +80 |
| CCP-20CF₃ | 10,0 % | Viskosität 20 °C | - |
| CCP-30CF₃ | 12,0 % | Δn (589 nm, 20 °C) | +0,0756 |
| CCP-40CF₃ | 8,0 % | n_{ε} (589 nm, 20 °C) | 1,5489 |
| CCP-50CF₃ | 12,0 % | V_{(10,0,20)} | 1,66 |
| ECP-3F.F | 12,0 % | V_{(50,0,20)} | 2,12 |
| CCP-3F.F.F | 14,0 % | V_{(90,0,20)} | 2,73 |
| CCP-5F.F.F | 12,0 % | | |

| Beispiel C | | | |
|---|---|---|---|
| PCH-5F | 12,0 % | S → N [°C] | - |
| PCH-7F | 7,0 % | Klärpunkt [°C] | +78 |
| CCP-20CF₃ | 10,0 % | Viskosität 20 °C | 16 |
| CCP-30CF₃ | 12,0 % | Δn (589 nm, 20 °C) | 0,0844 |
| CCP-40CF₃ | 8,0 % | n_{ε} (589 nm, 20 °C) | 1,5610 |
| CCP-50CF₃ | 12,0 % | V_{(10,0,20)} | 1,61 |
| BCH-3F.F | 8,0 % | V_{(50,0,20)} | 2,06 |
| BCH-5F.F | 6,0 % | V_{(90,0,20)} | 2,72 |
| CCP-3F.F.F | 13,0 % | | |
| CCP-5F.F.F | 12,0 % | | |

| Beispiel D | | | |
|---|---|---|---|
| PCH-5F | 12,0 % | S → N [°C] | - |
| PCH-7F | 8,0 % | Klärpunkt [°C] | +77 |
| CCP-20CF₃ | 10,0 % | Viskosität 20 °C | - |
| CCP-30CF₃ | 12,0 % | Δn (589 nm, 20 °C) | +0,0847 |
| CCP-40CF₃ | 8,0 % | n_{ε} (589 nm, 20 °C) | 1,5605 |
| CCP-50CF₃ | 12,0 % | V_{(10,0,20)} | 1,59 |
| BCH-3F.F.F | 14,0 % | V_{(50,0,20)} | - |
| ECCP-3F.F | 12,0 % | V_{(90,0,20)} | - |
| CCP-5F.F.F | 12,0 % | | |

| Beispiel E | | | |
|---|---|---|---|
| PCH-5F | 11,0 % | S → N [°C] | - |
| PCH-7F | 9,0 % | Klärpunkt [°C] | +75 |
| CCP-20CF₃ | 8,0 % | Viskosität 20 °C | - |
| CCP-30CF₃ | 10,0 % | Δn (589 nm, 20 °C) | 0,0876 |
| CCP-40CF₃ | 7,0 % | n_{ε} (589 nm, 20 °C) | 1,5666 |
| CCP-50CF₃ | 10,0 % | V_{(10,0,20)} | 1,51 |
| BCH-3F.F | 10,0 % | V_{(50,0,20)} | 1,95 |
| BCH-5F.F | 10,0 % | V_{(90,0,20)} | 2,54 |
| CCP-3F.F.F | 13,0 % | | |
| CCP-5F.F.F | 12,0 % | | |

| Beispiel F | | | |
|---|---|---|---|
| PCH-5F | 12,0 % | S → N [°C] | - |
| PCH-7F | 7,0 % | Klärpunkt [°C] | +76 |
| CCP-20CF₃ | 10,0 % | Viskosität 20 °C | - |
| CCP-30CF₃ | 12,0 % | Δn (589 nm, 20 °C) | 0,0835 |
| CCP-40CF₃ | 8,0 % | n_{ε} (589 nm, 20 °C) | 1,5595 |
| CCP-50CF₃ | 12,0 % | V_{(10,0,20)} | 1,49 |
| BCH-3F.F.F | 8,0 % | V_{(50,0,20)} | 1,93 |
| BCH-5F.F | 6,0 % | V_{(90,0,20)} | 2,52 |
| CCP-3F.F.F | 13,0 % | | |
| CCP-5F.F.F | 12,0 % | | |

| Beispiel G | | | |
|---|---|---|---|
| PCH-5F | 8,0 % | S → N [°C] | - |
| PCH-7F | 4,0 % | Klärpunkt [°C] | +81 |
| CCP-20CF₃ | 10,0 % | Viskosität 20 °C | - |
| CCP-30CF₃ | 12,0 % | Δn (589 nm, 20 °C) | +0,0907 |
| CCP-40CF₃ | 8,0 % | n_{ε} (589 nm, 20 °C) | 1,5672 |
| CCP-50CF₃ | 12,0 % | V_{(10,0,20)} | 1,46 |
| BCH-3F.F.F | 14,0 % | V_{(50,0,20)} | - |
| BCH-5F.F.F | 10,0 % | V_{(90,0,20)} | - |
| CCP-3F.F.F | 10,0 % | | |
| CCP-5F.F.F | 8,0 % | | |
| ECCP-3F.F | 4,0 % | | |

| Beispiel H | | | |
|---|---|---|---|
| PCH-5F | 8,0 % | S → N [°C] | - |
| CCP-20CF₃ | 10,0 % | Klärpunkt [°C] | +86 |
| CCP-30CF₃ | 12,0 % | Viskosität 20 °C | - |
| CCP-40CF₃ | 8,0 % | Δn (589 nm, 20 °C) | +0,0930 |
| CCP-50CF₃ | 12,0 % | n_{ε} (589 nm, 20 °C) | 1,5697 |
| BCH-3F.F.F | 14,0 % | V_{(10,0,20)} | 1,45 |
| BCH-5F.F.F | 11,0 % | V_{(50,0,20)} | 1,89 |
| CCP-3F.F.F | 12,0 % | V_{(90,0,20)} | 2,46 |
| CCP-5F.F.F | 7,0 % | | |
| ECCP-3F.F | 6,0 % | | |

## Patentansprüche

1. Fluorbenzolderivate der Formel I 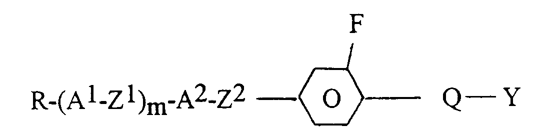 worin
R H, einen unsubstituierten, einen einfach durch CN oder CF₃ oder einen mindestens einfach durch Halogen substituierten Alkyl- oder Alkenylrest mit 1 bis 15 C-Atomen wobei in diesen Resten auch eine oder mehrere CH₂-Gruppen jeweils unabhängig voneinander durch -O-, -S-, 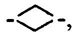 -CO-, -CO-O-, -O-CO-oder -O-CO-O- so ersetzt sein können, daß O-Atome nicht direkt miteinander verknüpft sind,
A¹ einen
(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo (2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) durch CN oder Fluor substituiert sein können,
Z¹ -CO-O-, -O-CO-, -CH₂O-, -OCH₂-, -CH₂CH₂-, -CH=CH.-, -C≡C-, eine Einfachbindung, -(CH₂)₄- oder -CH=CH-CH₂CH₂-,
m 0, 1 oder 2,
Y F oder Cl,
Q -CF₂-, -OCF₂- oder -OCHF-, und
A²-Z² 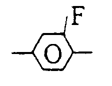 bedeutet.

2. Fluorbenzolderivate nach Anspruch 1, dadurch gekennzeichnet, daß der Rest Z¹ -(CH₂)₄- oder -CH=CH-CH₂CH₂- bedeutet.

3. Fluorbenzolderivate nach Anspruch 1, dadurch gekennzeichnet, daß A¹ 3-Fluor-1,4-phenylen oder 3,5-Difluor-1,4-phenylen bedeutet.

4. Fluorbenzolderivate nach Anspruch 1, dadurch gekennzeichnet, daß R Vinyl, Prop-1-enyl, But-1-enyl, But-3-enyl, Pent-1-enyl oder Pent-3-enyl bedeutet.

5. Fluorbenzolderivate nach Anspruch 1 , dadurch gekennzeichnet, daß m 0 bedeutet.

6. Fluorbenzolderivate nach Anspruch 1 , dadurch gekennzeichnet, daß m = 1 ist.

7. Fluorbenzolderivate nach Anspruch 1, gekennzeichnet durch die Formel 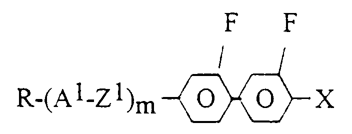 worin X OCHF₂, OCF₃ oder OCF₂Cl bedeutet und R, A¹, Z¹ und m die angegebenen Bedeutungen haben.

8. Verwendung von Verbindungen der Formel I als Komponenten flüssigkristalliner Medien.

9. Flüssigkistallines Medium mit mindestens zwei flüssigkristallinen Komponenten, dadurch gekennzeichnet, daß sie mindestens eine Verbindung der Formel I enthält.

10. Flüssigkristall-Anzeigeelement, dadurch gekennzeichnet, daß es ein flüssigkristallines Medium nach Anspruch 9 enthält.

11. Elektrooptisches Anzeigeelement, dadurch gekennzeichnet, daß es als Dielektrikum ein flüssigkristallines Medium nach Anspruch 9 enthält.

## Claims

1. Fluorobenzene derivatives of the formula I 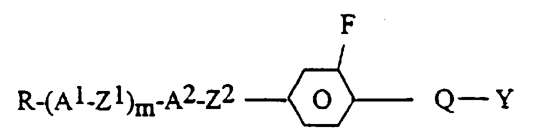 in which
R is H, an alkyl or alkenyl radical of 1 to 15 carbon atoms which is unsubstituted or mono-substituted by CN or CF₃ or at least mono-substituted by halogen, it being possible for one or more CH₂ groups in these radicals also to be replaced, in each case independently of one another, by -O-, -S-, 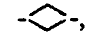 -CO-, -CO-O-, -O-CO- or -O-CO-O- in such a manner that O atoms are not linked directly to one another,
A¹ is a
(a) trans-1,4-cyclohexylene radical, in which one or more non-adjacent CH₂ groups can also be replaced by -O- and/or -S-,
(b) 1,4-phenylene radical, in which one or two CH groups can also be replaced by N,
(c) radical from the group comprising 1,4-cyclohexenylene, 1,4-bicyclo(2.2.2)-octylene, piperidine-1,4-diyl, naphthalene-2,6-diyl, decahydronaphthalene-2,6-diyl and 1,2,3,4-tetrahydronaphthalene-2,6-diyl,
it being possible for the radicals (a) and (b) to be substituted by CN or fluorine,
Z¹ is -CO-O-, -O-CO-, -CH₂O-, -OCH₂-, -CH₂CH₂-, -CH=CH-, -C≡C-, a single bond, -(CH₂)₄- or -CH=CH-CH₂CH₂-,
m is 0, 1 or 2,
Y is F or Cl,
Q is -CF₂-, -OCF₂- or -OCHF-, and
A²-Z² is

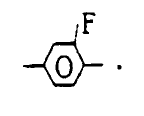

2. Fluorobenzene derivatives according to Claim 1, characterized in that the radical Z¹ is -(CH₂)₄- or -CH=CH-CH₂CH₂-.

3. Fluorobenzene derivatives according to Claim 1, characterized in that A¹ is 3-fluoro-1,4-phenylene or 3,5-difluoro-1,4-phenylene.

4. Fluorobenzene derivatives according to Claim 1, characterized in that R is vinyl, prop-1-enyl, but-1-enyl, but-3-enyl, pent-1-enyl or pent-3-enyl.

5. Fluorobenzene derivatives according to Claim 1, characterized in that m is 0.

6. Fluorobenzene derivatives according to Claim 1, characterized in that m is 1.

7. Fluorobenzene derivatives according to Claim 1, characterized by the formula 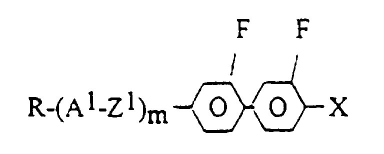 in which X is OCHF₂, OCF₃ or OCF₂Cl and R, A¹, Z¹ and m have the meanings indicated.

8. Use of compounds of the formula I as components of liquid-crystalline media.

9. Liquid-crystalline medium containing at least two liquid-crystalline components, characterized in that it contains at least one compound of the formula I.

10. Liquid-crystal display element, characterized in that it contains a liquid-crystalline medium according to Claim 9.

11. Electrooptic display element, characterized in that it contains, as dielectric, a liquid-crystalline medium according to Claim 9.

## Revendications

1. Dérivés de fluorobenzène de la formule I : 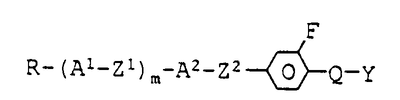 dans laquelle
R est H, un résidu alkyle ou alcényle ayant de 1 à 15 atomes de carbone, non substitué, monosubstitué par CN ou CF₃ ou au moins monosubstitué par un halogène, un ou plusieurs groupes CH₂ dans ces résidus pouvant également être remplacés indépendamment les uns des autres par ―O-, 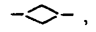 -S-, -CO-, -CO-O-, -O-CO- ou ―O-CO-O- de telle sorte que des atomes d'oxygène ne sont pas reliés directement les uns avec les autres.
A¹ est
(a) un résidu trans-1,4-cyclohexylène dans lequel un ou plusieurs groupes CH₂ non voisins peuvent également être remplacés par ―O- et/ou ―S-,
(b) un résidu 1,4-phénylène, dans lequel un ou deux groupes CH peuvent également être remplacés par N,
(c) un résidu choisi dans le groupe comprenant le 1,4-cyclohexénylène, le 1,4-bicyclo(2,2,2)-octylène, le pipéridin-1,4-diyle, le naphtalin-2,6-diyle, le décahydronaphtalin-2,6-diyle et le 1,2,3,4-tétrahydronaphtalin-2,6-diyle,
les résidus (a) et (b) pouvant être substitués par CN ou un fluor,
Z¹ est ―CO-O-, -O-CO-, -CH₂O-, -OCH₂-, -CH₂CH₂-, -CH=CH-,-C≡C-, une liaison simple, -(CH₂)₄- ou -CH=CH-CH₂CH₂-,
m vaut 0, 1 ou 2,
Y est F ou Cl,
Q est ―CF₂-, -OCF₂- ou ―OCHF-, et
A²-Z² est

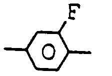

2. Dérivés de fluorobenzène selon la revendication 1, caractérisés en ce que le résidu Z¹ est -(CH₂)₄- ou -CH=CH-CH₂CH₂-.

3. Dérivés de fluorobenzène selon la revendication 1, caractérisés en ce que A¹ est un 3-fluoro-1,4-phénylène ou 3,5-difluoro-1,4-phénylène.

4. Dérivés de fluorobenzène selon la revendication 1, caractérisés en ce que R est un vinyle, 1-propényle, 1-butényle, 3-butényle, 1-pentényle ou 3-pentényle.

5. Dérivés de fluorobenzène selon la revendication 1, caractérisés en ce que m vaut 0.

6. Dérivés de fluorobenzène selon la revendication 1, caractérisés en ce que m vaut 1.

7. Dérivés de fluorobenzène selon la revendication 1, caractérisés par la formule : 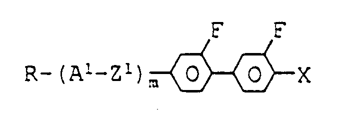 dans laquelle X est OCHF₂, OCF₃ ou OCF₂Cl et R, A¹, Z¹ et m ont les significations indiquées.

8. Utilisation de composés de la formule I comme composants de milieux à cristaux liquides.

9. Milieu à cristaux liquides avec au moins deux composants à cristaux liquides, caractérisé en ce qu'il contient au moins un composé de la formule I.

10. Elément indicateur à cristaux liquides, caractérisé en ce qu'il contient un milieu à cristaux liquides selon la revendication 9.

11. Elément indicateur électro-optique, caractérisé en ce qu'il contient comme diélectrique un milieu à cristaux liquides selon la revendication 9.
